# EUROPEAN PATENT APPLICATION

(11) **EP 4 553 087 A1**
(43) Date of publication of application: **14.05.2025**
(21) Application number: 23835831.1
(22) Date of filing: 05.07.2023
(51) Int. Cl.: C07K 16/30, A61P 35/00, G01N 33/574, A61K 39/00

(54) **NOVEL TCTP-BINDING MOLECULE**

(30) Priority: 07.07.2022 KR 20220083693
(71) Applicant: Boostimmune, Inc., Seoul 06736 (KR)
(72) Inventor: LEE, Gwanghee, Seoul 06736 (KR); RYOU, Jeong-Hyun, Seoul 06736 (KR); JEON, Hyungsu, Seoul 06736 (KR); KIM, Won-Tae, Seoul 06736 (KR)
(74) Representative: ABG Intellectual Property Law, S.L.
(86) International application number: PCT/KR2023/009510
(87) International publication number: WO 2024/010365

(57) **Abstract**

The present invention relates to a novel binding molecule or a fragment thereof, which specifically binds to an extracellular translationally controlled tumor protein (TCTP). The present invention relates to such a binding molecule or a fragment thereof, and use thereof. The binding molecule and the fragment thereof according to the present invention can be used to diagnose, treat, or prevent cancer. The binding molecule and the fragment thereof according to the present invention can be used to alleviate or prevent immune suppression in a tumor immune microenvironment. The binding molecule and the fragment thereof according to the present invention can be used to diagnose, treat, or prevent cancer in a patient who has no response, has a limited response, or is likely to have such a response to treatment using a cancer immunotherapeutic agent.

## Description

### TECHNICAL FIELD

The present invention relates to a novel binding molecule or a fragment thereof, which specifically binds to an extracellular translationally controlled tumor protein (TCTP). The binding molecule and the fragment thereof according to the present invention can be used to diagnose, treat, or prevent cancer. The binding molecule and the fragment thereof according to the present invention can be used to alleviate or prevent immune suppression in a tumor immune microenvironment. The binding molecule and the fragment thereof according to the present invention can be used to diagnose, treat, or prevent cancer in a patient who has no response, has a limited response, or is likely to have such a response to treatment using a cancer immunotherapeutic agent.

### BACKGROUND ART

The tumor immune microenvironment ("TIME") promotes or inhibits the growth of tumor cells through various interactions that occur between tumor cells and various cells surrounding the tumor cells. There are different types of cells in the tumor immune microenvironment, which include various fibroblasts, endothelial cells, mast cells, and immune cells. Examples of the immune cell present in the tumor immune microenvironment include a T lymphocyte, a natural killer (NK) cell, a tumor-associated macrophage (TAM), a dendritic cell (DC), a bone marrow-derived suppressor cell (MDSC), a regulatory T cell (Treg), and a neutrophil. In the tumor immune microenvironment, there is a mechanism that suppresses the immune response against tumor cells, and this is known to be the main cause of limiting the efficacy of the immune anticancer therapy.

TCTP is a highly conserved protein in eukaryotes. TCTP is located in both the cytoplasm and the nucleus and is expressed in various tissues, and it is regulated in response to a wide range of extracellular stimuli. TCTP is also found as a dimer and is known to interact with other proteins including MCL1 and p53 (for example, Acunzo et al. (2014) Cancer treatment reviews 40.6: 760-769). It has been reported that dimers of TCTP can be formed between intact TCTPs or N-terminal cleaved forms thereof (Kim et al. (2009): PloS one 4.7: e6464). Treatment strategies to suppress the expression of TCTP in cells by using nucleic acid molecules targeting mRNAs that encode TCTP, such as antisense oligonucleotides, siRNA, and shRNA, have been proposed (for example, WO 2012/080509). However, the function of TCTP outside the cells has not yet been established.

### DISCLOSURE OF THE INVENTION

### TECHNICAL PROBLEM

The inventors of the present invention aimed to provide a novel binding molecule that specifically binds to TCTP based on the discovery that TCTP is released from tumor cells and then the released TCTP acts as an immunoregulatory factor that regulates the function or balance (aspects such as number or lifespan of cells) of immune cells such as T cells, NK cells, and/or MDSCs in the tumor immune microenvironment. The novel binding molecules according to the present invention can inhibit the function of suppressive immune cells in the tumor immune microenvironment and/or activate the function of anti-cancer immune cells by specifically binding to extracellular TCTP and suppressing the function thereof outside the cell.

### TECHNICAL SOLUTION

The present invention provides a novel binding molecule or a fragment thereof, which specifically binds to extracellular TCTP.

The present invention provides a binding molecule or a fragment thereof, which specifically binds to human TCTP, with a dissociation constant (Kd) of about 1 × 10⁻⁷ M or less or 5 × 10⁻⁸ M or less in a case of being measured by surface plasmon resonance. The binding molecule or the fragment thereof can suppress a function of suppressive immune cells and/or activate anti-cancer immune cells by binding to TCP or a cleaved form thereof, or a multimer thereof to suppress a function thereof outside the cells.

The present invention provides a binding molecule or a fragment thereof, which specifically binds to TCTP, a post-translational variant thereof, a cleaved form thereof, or a multimer thereof, which comprises one or more of the heavy chain CDR1, the heavy chain CDR2, the heavy chain CDR3, the light chain CDR1, the light chain CDR2, and the light chain CDR3, which are as defined in the following (a) to (f), respectively.
(a) The heavy chain CDR1 comprises
   (a1) an amino acid sequence of X1-X2-X3-X4-H (where X1 is S, G, or V, X2 is Y, H, or N, X3 is A or Y, and X4 is M or V),
   (a2) an amino acid sequence of S-X5-X6-X7-X8-W-X9 (where X5 is S or N, X6 is N or S, X7 is A or W, X8 is A or is not present, and X9 is N or S),
   (a3) an amino acid sequence of G-Y-T-F-T-G-Y-M-H, or
   (a4) an amino acid sequence of N-A-R-M-G-V-S, or
   a conservative amino acid substituent thereof;
(b) the heavy chain CDR2 comprises
   (b1) an amino acid sequence of E-I-X1-H-X2-G-X3-T-Y-N-P-S-L-K-S (where X1 is D or Y, X2 is S or is not present, and X3 is T or V),
   (b2) an amino acid sequence of W-I-N-X4-X5-X6-X7-X8-T-X9-Y-X10-Q-K-F-Q-X11 (where X4 is A or P, X5 is G, I, or N, X6 is K, N, S, or T, X7 is G or S, X8 is G, N, Y, D, or K, X9 is K, N, or E, X10 is A or S, and X11 is D or G),
   (b3) an amino acid sequence of R-T-X12-Y-X13-S-W-Y-N-D-Y-A-X14-S-V-K-S (where X12 is F or Y, X13 is K or R, and X14 is E or V), or
   (b4) an amino acid sequence of H-I-F-S-N-D-E-K-S-Y-S-Y-S-L-K-S, or
   a conservative amino acid substituent thereof;
(c) the heavy chain CDR3 comprises
   (c1) an amino acid sequence of G-X1-X2-X3-X4-F-D-X5 (where X1 is R or G, X2 is A or S, X3 is A or V, X4 is A or V, and X5 is P or Y),
   (c2) an amino acid sequence of D-G-S-G-S-Y-E-G-Y,
   (c3) an amino acid sequence of W-V-F-D-Y,
   (c4) an amino acid sequence of X6-A-X7-R-L-X8-AX9-D-I (where X6 is L or P, X7 is P or W, X8 is G or M, and X9 is F or Y),
   (c5) an amino acid sequence of R-L-T-I-V-R-G-V-M-R-G-M-D-V,
   (c6) an amino acid sequence of G-Y-Y-D-I-L-T-G-Y-T-T-D-A-F-D-I,
   (c7) an amino acid sequence of X10-X11-X12-X13-X14-X15-X16-X17-X18-F-D-Y (where X10 is V or is not present, X11 is R or L or is not present, X12 is G or L, X13 is Y, I, T, or W, X14 is F or T, X15 is D or G, X16 is W, T, Y, or E, X17 is W, T, Y, or L, and X18 is A, P, or Y),
   (c8) an amino acid sequence of G-G-V-L-Y-F-G-E-L-S-R-F-D-Y, or
   (c9) an amino acid sequence of E-G-I-T-V-G-R-G-M-L-Y-F-D-L, or
   a conservative amino acid substituent thereof;
(d) the light chain CDR1 comprises
   (d1) an amino acid sequence of R-A-S-Q-X1-X2-X3-X4-X5-L-X6 (where X1 is A, D, or G, X2 is I or V, X3 is S, R, or G, X4 is N or S, X5 is H, Y, D, N, or S, and X6 is A, G, or H),
   (d2) an amino acid sequence of X7-G-X8-X9-S-X10-X11-G-X12-X13-X14-X15-V-S (where X7 is S or T, X8 is S or T, X9 is N or S, X10 is D or N, X11 is I or V, X12 is G, N, or T, X13 is F, K, or Y, X14 is N or is not present, and X15 is K or Y),
   (d3) an amino acid sequence of K-S-S-Q-X16-X17-L-Y-X18-X19-N-N-K-X20-Y-X21-A (where X16 is N or S, X17 is I, L, or V, X18 is N or S, X19 is A or S, X20 is D or N, and X21 is I or L), or
   (d4) an amino acid sequence of R-S-S-Q-S-V-X22-X23-D-G-N-T-X24-L-X25 (where X22 is H or Y, X23 is R or S, X24 is Y or H, and X25 is S, N, or K), or
   a conservative amino acid substituent thereof;
(e) the light chain CDR2 comprises an amino acid sequence of X1-X2-X3-X4-X5-X6-X7 (where X1 is A, D, E, G, K, W, or Y, X2 is A, D, I, or V, X3 is N, S, or T, X4 is I, K, N, Q, S, or T, X5 is L, R, S, or W, X6 is A, D, F, G, K, P, Q, or Y, and X7 is F, S or T) or a conservative amino acid substituent thereof; and
(f) the light chain CDR3 comprises
   (f1) an amino acid sequence of X1-Q-X2-X3-X4-X5-P-X6-T (where X1 is M or Q, X2 is A, G, or Y, X3 is F, H, N, S, or Y, X4 is G, H, Q, N, S, or Y, X5 is F, I, L, T, Y, or W, and X6 is H, I, L, P, R, W, or Y),
   (f2) an amino acid sequence of X7-X8-X9-X10-X11-X12-X13-X14-X15-V (where X7 is A, N, or V, X8 is S or T, X9 is W or Y, X10 is A or D, X11 is G or S, X12 is D, N, or S, X13 is L or N, X14 is N or R, and X15 is A or L), or
   (f3) an amino acid sequence of X16-Q-X17-X18-S-X19-P-X20-T (where X16 is L or H, X17 is H or S, X18 is N or S, X19 is Y or L, and X20 is Y, R, or Q), or
   a conservative amino acid substituent thereof.

### BRIEF DESCRIPTION OF THE DRAWINGS

Figs. 1a and 1b show the ELISA test results for assaying the binding of anti-TCTP antibodies to human TCTP.
Fig. 2 shows the results obtained by assaying the binding capacity of mutants of an anti-TCTP Ab10 antibody to mouse TCTP and human TCTP through ELISA.
Fig. 3a shows the ELISA test results showing that anti-TCTP antibodies inhibit TCTP-TLR2 binding.
Fig. 3b shows the test results of the HEK Blue^{™} hTLR2 reporter cell, which shows that anti-TCTP antibodies inhibit TLR2 signaling.
Fig. 4a to Fig. 4f are representative plots showing FACS analysis of a single cell suspension obtained from HT29 tumors that have been subcutaneously transplanted into BALB/c nude mice that do not have T cells.
Fig. 5 shows the results obtained by measuring the level of PMN-MDSC through FACS analysis in a case where HT29 tumors that have been subcutaneously transplanted into BALB/c nude mice that do not have T cells are treated with anti-TCTP antibodies.
Fig. 6a shows changes in tumor volume over time in a case where SL4 cells which have been subjected to knock-in with human TCTP are treated with anti-TCTP antibodies alone.
Figs. 6b to 6e show changes in tumor volume over time in a case where four kinds of cancer cells (Pan02, Hepa1-6, B16BL6, and MC38) different from each other are treated with an anti-TCTP antibody or an anti-PD-1 antibody.
Fig. 7 shows changes in tumor volume over time in a case where B16F10 cells are treated with each of an anti-PD-1 antibody and an anti-TCTP antibody alone or in combination.
Fig. 8 shows changes in tumor volume over time in a case where SL4 cells are treated with each of an anti-CTLA-4 antibody and an anti-TCTP antibody alone or in combination.

### MODE FOR CARRYING OUT THE INVENTION

The present invention relates to a binding molecule or a fragment thereof, which inhibits the function of suppressive immune cells in the tumor immune microenvironment and/or activates the function of anti-cancer immune cells by specifically binding to extracellular TCTP and suppressing the function thereof outside the cell.

The "extracellular" described above preferably refers to a tumor immune microenvironment. According to previous research according to the present invention, it was confirmed that TCTP is released from tumor cells, particularly tumor cells damaged or dead under stress conditions. Accordingly, the "extracellular" may mean that the release has occurred from such tumor cells; however, the tumor cells are not necessarily limited to damaged or dead cells.

Tumors are known to exert various strategies to slow down immune responses that can interfere with the proliferation of the tumors themselves, including the activation of suppressive immune cells in the tumor immune microenvironment, the suppression of anti-cancer immune cells, or the upregulation of immunosuppressive cytokines and chemokines. Examples of the suppressive immune cell include a tumor-associated macrophage (TAM), a bone marrow-derived suppressor cell (MDSC), a tumor-associated neutrophil (TAN), a cancer-associated fibroblast (CAF), and a regulatory T cell (Treg). Examples of the anti-cancer immune cell in the tumor immune microenvironment include, but are not limited to, a T cell and an NK cell. Examples of the immunosuppressive cytokine and chemokine which are secreted into the tumor immune microenvironment include CCL2, CCL3, CCL5, CCL17, CCL19, CCL21, CCL22, CCL28, CXCL5, CXCL8, CXCL12, CXCL15, CXCL17, MIF, HMGB1, CSF, CSF2, IL-4, IL-6, IL-10, IL-13, G-CSF, PGE2, and TGF-β. In the present specification, the fact that immune suppression is alleviated or suppressed in the tumor immune microenvironment means that the activity of suppressive immune cells in the tumor immune microenvironment is reduced, suppressed, or inhibited, and/or the activity of anti-cancer immune cells is increased, enhanced, or activated, and/or the activity of immunosuppressive cytokines or chemokines is downregulated.

The "function outside the cell" regarding TCTP may be, for example, a function of binding to an immunoregulatory receptor (for example, TLR2) present on a suppressive immune cell (for example, a bone marrow-derived immunosuppressive cell), or a function of inducing secretion of cytokines (for example, CXCL1 and CXCL2) from a suppressive immune cell (for example, a bone marrow-derived immunosuppressive cell). As a result of the suppression of such functions of TCTP outside the cell, the function of the suppressive immune cell can be suppressed. The fact that the function of the suppressive immune cell is suppressed means that the immunosuppressive activity of the suppressive immune cell is directly or indirectly reduced, suppressed, or inhibited. Such reduction, suppression, or inhibition of immunosuppressive activity may mean that the accumulation or recruitment of suppressive immune cells in the tumor immune microenvironment is reduced, suppressed, or inhibited. The reduction, suppression, or inhibition of the immunosuppressive activity of the suppressive immune cell may be exhibited, for example, in the following ways: the induction of death of the suppressive immune cell, the reduction of survival rate of the suppressive immune cell, the reduction of secretion of the anti-cancer immunosuppressive cytokine by the suppressive immune cell, the reduction of secretion of the T-cell inhibitory substance by the suppressive immune cell, the reduction of differentiation into the suppressive immune cell, and the suppression of exhibition of the immune checkpoint by the suppressive immune cell.

As described above, the effect of inhibiting the function of suppressive immune cells in the tumor immune microenvironment and/or activating the function of anti-cancer immune cells by specifically binding to extracellular TCTP and suppressing the function thereof outside the cell can be achieved by the TCTP-specific binding molecule or the fragment thereof according to the present invention as described below.

The binding molecule or the fragment thereof according to the present invention can suppress a function of a suppressive immune cell and/or activate an anti-cancer immune cell by binding to human TCIP, a cleaved form thereof, or a multimer thereof with a dissociation constant (Kd) of about 1 × 10⁻⁷ M or less or 5 × 10⁻⁸ M or less in a case of being measured by surface plasmon resonance, and then suppress a function thereof outside the cell.

The term "surface plasmon resonance" used in the present specification refers to, for example, an optical phenomenon that enables analysis of interactions, which is carried out by detecting protein concentrations in a biosensor matrix using a BIA Core^{™} system.

In addition, the binding molecule or the fragment thereof according to the present invention may comprise one or more of the heavy chain CDR1, the heavy chain CDR2, the heavy chain CDR3, the light chain CDR1, the light chain CDR2, and the light chain CDR3, which are as defined in the following (a) to (f), respectively.
(a) The heavy chain CDR1 comprises
   (a1) an amino acid sequence of X1-X2-X3-X4-H (where X1 is S, G, or V, X2 is Y, H, or N, X3 is A or Y, and X4 is M or V),
   (a2) an amino acid sequence of S-X5-X6-X7-X8-W-X9 (where X5 is S or N, X6 is N or S, X7 is A or W, X8 is A or is not present, and X9 is N or S),
   (a3) an amino acid sequence of G-Y-T-F-T-G-Y-M-H, or
   (a4) an amino acid sequence of N-A-R-M-G-V-S, or
   a conservative amino acid substituent thereof;
(b) the heavy chain CDR2 comprises
   (b1) an amino acid sequence of E-I-X1-H-X2-G-X3-T-Y-N-P-S-L-K-S (where X1 is D or Y, X2 is S or is not present, and X3 is T or V),
   (b2) an amino acid sequence of W-I-N-X4-X5-X6-X7-X8-T-X9-Y-X10-Q-K-F-Q-X11 (where X4 is A or P, X5 is G, I, or N, X6 is K, N, S, or T, X7 is G or S, X8 is G, N, Y, D, or K, X9 is K, N, or E, X10 is A or S, and X11 is D or G),
   (b3) an amino acid sequence of R-T-X12-Y-X13-S-W-Y-N-D-Y-A-X14-S-V-K-S (where X12 is F or Y, X13 is K or R, and X14 is E or V), or
   (b4) an amino acid sequence of H-I-F-S-N-D-E-K-S-Y-S-Y-S-L-K-S, or
   a conservative amino acid substituent thereof;
(c) the heavy chain CDR3 comprises
   (c1) an amino acid sequence of G-X1-X2-X3-X4-F-D-X5 (where X1 is R or G, X2 is A or S, X3 is A or V, X4 is A or V, and X5 is P or Y),
   (c2) an amino acid sequence of D-G-S-G-S-Y-E-G-Y,
   (c3) an amino acid sequence of W-V-F-D-Y,
   (c4) an amino acid sequence of X6-A-X7-R-L-X8-AX9-D-I (where X6 is L or P, X7 is P or W, X8 is G or M, and X9 is F or Y),
   (c5) an amino acid sequence of R-L-T-I-V-R-G-V-M-R-G-M-D-V,
   (c6) an amino acid sequence of G-Y-Y-D-I-L-T-G-Y-T-T-D-A-F-D-I,
   (c7) an amino acid sequence of X10-X11-X12-X13-X14-X15-X16-X17-X18-F-D-Y (where X10 is V or is not present, X11 is R or L or is not present, X12 is G or L, X13 is Y, I, T, or W, X14 is F or T, X15 is D or G, X16 is W, T, Y, or E, X17 is W, T, Y, or L, and X18 is A, P, or Y),
   (c8) an amino acid sequence of G-G-V-L-Y-F-G-E-L-S-R-F-D-Y, or
   (c9) an amino acid sequence of E-G-I-T-V-G-R-G-M-L-Y-F-D-L, or
   a conservative amino acid substituent thereof;
(d) the light chain CDR1 comprises
   (d1) an amino acid sequence of R-A-S-Q-X1-X2-X3-X4-X5-L-X6 (where X1 is A, D, or G, X2 is I or V, X3 is S, R, or G, X4 is N or S, X5 is H, Y, D, N, or S, and X6 is A, G, or H),
   (d2) an amino acid sequence of X7-G-X8-X9-S-X10-X11-G-X12-X13-X14-X15-V-S (where X7 is S or T, X8 is S or T, X9 is N or S, X10 is D or N, X11 is I or V, X12 is G, N, or T, X13 is F, K, or Y, X14 is N or is not present, and X15 is K or Y),
   (d3) an amino acid sequence of K-S-S-Q-X16-X17-L-Y-X18-X19-N-N-K-X20-Y-X21-A (where X16 is N or S, X17 is I, L, or V, X18 is N or S, X19 is A or S, X20 is D or N, and X21 is I or L), or
   (d4) an amino acid sequence of R-S-S-Q-S-V-X22-X23-D-G-N-T-X24-L-X25 (where X22 is H or Y, X23 is R or S, X24 is Y or H, and X25 is S, N, or K), or
   a conservative amino acid substituent thereof;
(e) the light chain CDR2 comprises
   an amino acid sequence of X1-X2-X3-X4-X5-X6-X7 (where X1 is A, D, E, G, K, W, or Y, X2 is A, D, I, or V, X3 is N, S, or T, X4 is I, K, N, Q, S, or T, X5 is L, R, S, or W, X6 is A, D, F, G, K, P, Q, or Y, and X7 is F, S or T) or a conservative amino acid substituent thereof; and
(f) the light chain CDR3 comprises
   (f1) an amino acid sequence of X1-Q-X2-X3-X4-X5-P-X6-T (where X1 is M or Q, X2 is A, G, or Y, X3 is F, H, N, S, or Y, X4 is G, H, Q, N, S, or Y, X5 is F, I, L, T, Y, or W, and X6 is H, I, L, P, R, W, or Y),
   (f2) an amino acid sequence of X7-X8-X9-X10-X11-X12-X13-X14-X15-V (where X7 is A, N, or V, X8 is S or T, X9 is W or Y, X10 is A or D, X11 is G or S, X12 is D, N, or S, X13 is L or N, X14 is N or R, and X15 is A or L), or
   (f3) an amino acid sequence of X16-Q-X17-X18-S-X19-P-X20-T (where X16 is L or H, X17 is H or S, X18 is N or S, X19 is Y or L, and X20 is Y, R, or Q), or
   a conservative amino acid substituent thereof.

The "conservative amino acid substitution" is a substitution of an amino acid residue by another amino acid residue having a side chain (R group) similar in chemical properties (for example, charge or hydrophobicity). In general, the conservative amino acid substitution does not substantially change the functional properties of proteins. In a case where two or more amino acid sequences differ from each other due to conservative substitutions, the degree of sequence identity or similarity in terms of % may be adjusted upwards to correct the conservative properties of the substitutions. The means for carrying out this adjustment are widely known publicly to those skilled in the art [see the literature, Pearson (1994) Methods Mol. Biol. 24: 307-331]. Examples of the amino acid group having a side chain similar in chemical properties include: (1) aliphatic side chains: glycine, alanine, valine, leucine, and isoleucine; (2) aliphatic-hydroxyl side chains: serine and threonine; (3) amide-containing side chains: asparagine and glutamine; (4) aromatic side chains: phenylalanine, tyrosine, and tryptophan; (5) basic side chains: lysine, arginine, and histidine; (6) acidic side chains: aspartate and glutamate; and (7) sulfurcontaining side chains: cysteine and methionine. Preferred conservative amino acid substitution groups are as follows. Valine-leucine-isoleucine, phenylalanine-tyrosine, lysinearginine, alanine-valine, glutamate-aspartate, and asparagineglutamine. Alternatively, the conservative substitution may be any change having a positive value in the PAM250 Log Odds Matrix described in the literature, Gonnet et al. (1992) Science 256: 1443-45.

The binding molecule or the fragment thereof according to the present invention may contain heavy chain CDR1, heavy chain CDR2, heavy chain CDR3, light chain CDR1, light chain CDR2, and light chain CDR3, which comprise amino acid sequences such as the following sequences or conservative amino acid substituents thereof.

**[Table 1]**

| CDR sequence of antibody Ab1 | | |
|---|---|---|
| Heavy chain CDR1 | Heavy chain CDR2 | Heavy chain CDR3 |
| GYYMH (sequence 1) | WINPNSGGTNYAQKFQG (sequence 2) | GITGTTPFDY (sequence 3) |
| Light chain CDR1 | Light chain CDR2 | Light chain CDR3 |
| RASQGIRNDLG (sequence 4) | AASSLQS (sequence 5) | LQHNSYPYT (sequence 6) |

**[Table 2]**

| CDR sequence of antibody Ab2 | | |
|---|---|---|
| Heavy chain CDR1 | Heavy chain CDR2 | Heavy chain CDR3 |
| NARMGVS (sequence 7) | HIFSNDEKSYSTSLKS (sequence 8) | VLLWFGELYFDY (sequence 9) |
| Light chain CDR1 | Light chain CDR2 | Light chain CDR3 |
| RASQSVSSNLA (sequence 10) | GASTRAT (sequence 11) | QQYNNWPRT (sequence 12) |

**[Table 3]**

| CDR sequence of antibody Ab3 | | |
|---|---|---|
| Heavy chain CDR1 | Heavy chain CDR2 | Heavy chain CDR3 |
| GYYMH (sequence 13) | WINPNSGGTNYAQKFQG (sequence 14) | LGTTGYYYFDY (sequence 15) |
| Light chain CDR1 | Light chain CDR2 | Light chain CDR3 |
| RASQSIGSSLH (sequence 16) | YASQSFS (sequence 17) | HQSSSLPRT (sequence 18) |

**[Table 4]**

| CDR sequence of antibody Ab4 | | |
|---|---|---|
| Heavy chain CDR1 | Heavy chain CDR2 | Heavy chain CDR3 |
| SNAMH (sequence 19) | WINAGNGNTKYSQFQG (sequence 20) | GYYDILTGYTTDAFDI (sequence 21) |
| Light chain CDR1 | Light chain CDR2 | Light chain CDR3 |
| RSSQSLVYSDGNTYLN (sequence 22) | KVSNRDS (sequence 23) | MQGTHWPYT (sequence 24) |

**[Table 5]**

| CDR sequence of antibody Ab5 | | |
|---|---|---|
| Heavy chain CDR1 | Heavy chain CDR2 | Heavy chain CDR3 |
| SYAMH (sequence 25) | WINAGNGYTEYSQKFQG (sequence 26) | RGYFDWWAFDY (sequence 27) |
| Light chain CDR1 | Light chain CDR2 | Light chain CDR3 |
| RASQSIGSSLH (sequence 28) | YASQSFS (sequence 29) | HQSSSLPQT (sequence 30) |

**[Table 6]**

| CDR sequence of antibody Ab6 | | |
|---|---|---|
| Heavy chain CDR1 | Heavy chain CDR2 | Heavy chain CDR3 |
| SSNWWS (sequence 31) | EIDHSGTTTYNPSLKS (sequence 32) | DGSGSYEGY (sequence 33) |
| Light chain CDR1 | Light chain CDR2 | Light chain CDR3 |
| RASQAISNHLA (sequence 34) | AASSLQS (sequence 35) | QQYHSYPIT (sequence 36) |

**[Table 7]**

| CDR sequence of antibody Ab7 | | |
|---|---|---|
| Heavy chain CDR1 | Heavy chain CDR2 | Heavy chain CDR3 |
| SSNWWS (sequence 37) | EIYHGVTTYNPSLKS (sequence 38) | DGSGSYEGY (sequence 39) |
| Light chain CDR1 | Light chain CDR2 | Light chain CDR3 |
| RASQDISNYLA (sequence 40) | AASSLQS (sequence 41) | QQYHYYPIT (sequence 42) |

**[Table 8]**

| CDR sequence of antibody Ab8 | | |
|---|---|---|
| Heavy chain CDR1 | Heavy chain CDR2 | Heavy chain CDR3 |
| SYAMH (sequence 43) | WINAGNGNTKYSQFQD (sequence 44) | WVFDY (sequence 45) |
| Light chain CDR1 | Light chain CDR2 | Light chain CDR3 |
| RSSQSLVHRDGNTYLS (sequence 46) | KISNRFF (sequence 47) | MQATQFPHT (sequence 48) |

**[Table 9]**

| CDR sequence of antibody Ab9 | | |
|---|---|---|
| Heavy chain CDR1 | Heavy chain CDR2 | Heavy chain CDR3 |
| SNSAAWN (sequence 49) | RTYYRSKWYNDYAVSVKS (sequence 50) | EGITVGRGVMLYFDL (sequence 51) |
| Light chain CDR1 | Light chain CDR2 | Light chain CDR3 |
| RSSQSLVYSDGNTHLK (sequence 52) | KVSKWDS (sequence 53) | MQGTHWPPT (sequence 54) |

**[Table 10]**

| CDR sequence of antibody Ab10 | | |
|---|---|---|
| Heavy chain CDR1 | Heavy chain CDR2 | Heavy chain CDR3 |
| GYTFTGYYMH (sequence 55) | WINPNSGGTNYAQKFQG (sequence 56) | PAPRLRGAFDI (sequence 57) |
| Light chain CDR1 | Light chain CDR2 | Light chain CDR3 |
| SGSSSNIGNKVS (sequence 58) | DDNKRPS (sequence 59) | ATWDSSLRAV (sequence 60) |

**[Table 11]**

| CDR sequence of antibody Ab12 | | |
|---|---|---|
| Heavy chain CDR1 | Heavy chain CDR2 | Heavy chain CDR3 |
| SHAVH (sequence 61) | WINAGKGNTKYSQFQG (sequence 62) | GRAVAFDP (sequence 63) |
| Light chain CDR1 | Light chain CDR2 | Light chain CDR3 |
| TGTNSDIGYNYVS (sequence 64) | EVNKRPS (sequence 65) | ASYAGDNNLV (sequence 66) |

**[Table 12]**

| CDR sequence of antibody Ab13 | | |
|---|---|---|
| Heavy chain CDR1 | Heavy chain CDR2 | Heavy chain CDR3 |
| SNSAAWN (sequence 67) | RTYYRSKWYNDYAVSVKS (sequence 68) | GGAAVFDY (sequence 69) |
| Light chain CDR1 | Light chain CDR2 | Light chain CDR3 |
| KSSQSILYNSNKNYLA (sequence 70) | WASTRGS (sequence 71) | QQYYYSTPLT (sequence 72) |

**[Table 13]**

| CDR sequence of antibody Ab14 | | |
|---|---|---|
| Heavy chain CDR1 | Heavy chain CDR2 | Heavy chain CDR3 |
| GYYMH (sequence 73) | WINPNSGDTNYAQKFQG (sequence 74) | GGVLLYFGELSRFDY (sequence 75) |
| Light chain CDR1 | Light chain CDR2 | Light chain CDR3 |
| TGTSSDVGGYNYVS (sequence 76) | EVTKRPS (sequence 77) | ASYAGDNNLV (sequence 78) |

**[Table 14]**

| CDR sequence of antibody Ab15 | | |
|---|---|---|
| Heavy chain CDR1 | Heavy chain CDR2 | Heavy chain CDR3 |
| VNAMH (sequence 79) | WINAGTGKTKYSQFQG (sequence 80) | GRAVAFDP (sequence 81) |
| Light chain CDR1 | Light chain CDR2 | Light chain CDR3 |
| TGTSSDVGGYDYVS (sequence 82) | EVNKRPS (sequence 83) | NSYAGNNNNLV (sequence 84) |

**[Table 15]**

| CDR sequence of antibody Ab16 | | |
|---|---|---|
| Heavy chain CDR1 | Heavy chain CDR2 | Heavy chain CDR3 |
| SNSAAWN (sequence 85) | RTYKSKWYNDYAVSVKS (sequence 86) | GGSAVFDY (sequence 87) |
| Light chain CDR1 | Light chain CDR2 | Light chain CDR3 |
| KSSQSILYSSNKNYLA (sequence 88) | WASTRKS (sequence 89) | QQYYGLPLT (sequence 90) |

**[Table 16]**

| CDR sequence of antibody Ab17 | | |
|---|---|---|
| Heavy chain CDR1 | Heavy chain CDR2 | Heavy chain CDR3 |
| SNSAAWN (sequence 91) | RTYKSKWYNDYAVSVKS (sequence 92) | GGSAVFDY (sequence 93) |
| Light chain CDR1 | Light chain CDR2 | Light chain CDR3 |
| KSSQSVLYSSNNKNYLA (sequence 94) | WASTRKS (sequence 95) | QQYYGIPLT (sequence 96) |

**[Table 17]**

| CDR sequence of antibody Ab18 | | |
|---|---|---|
| Heavy chain CDR1 | Heavy chain CDR2 | Heavy chain CDR3 |
| SNSAAWN (sequence 97) | RTYYRSKWYNDYAVSVKS (sequence 98) | GGAAVFDY (sequence 99) |
| Light chain CDR1 | Light chain CDR2 | Light chain CDR3 |
| KSSQSILYSSNKNYLA (sequence 100) | WASTRGS (sequence 101) | QQYYYSTPLT (sequence 102) |

**[Table 18]**

| CDR sequence of antibody Ab19 | | |
|---|---|---|
| Heavy chain CDR1 | Heavy chain CDR2 | Heavy chain CDR3 |
| SSSAAWN (sequence 103) | RTYYRSKWYNDYAESVKS (sequence 104) | RLTIVRGVMRGGMDV (sequence 105) |
| Light chain CDR1 | Light chain CDR2 | Light chain CDR3 |
| KSSQNLLYSANNKDYLA (sequence 106) | WASTRYS (sequence 107) | QQYYYSTPWT (sequence 108) |

**[Table 19]**

| CDR sequence of antibody Ab20 | | |
|---|---|---|
| Heavy chain CDR1 | Heavy chain CDR2 | Heavy chain CDR3 |
| SSSAAWN (sequence 109) | RTFYRSKWYNDYAESVKS (sequence 110) | RLTIVRGVMRGGMDV (sequence 111) |
| Light chain CDR1 | Light chain CDR2 | Light chain CDR3 |
| KSSQNLLYSANNKDYIA (sequence 112) | WASIRYS (sequence 113) | QQYFSTPWT (sequence 114) |

**[Table 20]**

| CDR sequence of antibody Ab21 | | |
|---|---|---|
| Heavy chain CDR1 | Heavy chain CDR2 | Heavy chain CDR3 |
| SSSAAWN (sequence 115) | RTYYRSKWYNDYAESVKS (sequence 116) | RLTIVRGVMRGGMDV (sequence 117) |
| Light chain CDR1 | Light chain CDR2 | Light chain CDR3 |
| KSSQNLLYSANNKDYLA (sequence 118) | WASTRYS (sequence 119) | QQYYYSTPWT (sequence 120) |

**[Table 21]**

| CDR sequence of antibody Ab22 | | |
|---|---|---|
| Heavy chain CDR1 | Heavy chain CDR2 | Heavy chain CDR3 |
| SSSAAWN (sequence 121) | RTYYRSKWYNDYAESVKS (sequence 122) | RLTIVRGVMRGGMDV (sequence 123) |
| Light chain CDR1 | Light chain CDR2 | Light chain CDR3 |
| KSSQNLLYSANNKDYLA (sequence 124) | WASTRYS (sequence 125) | QQYYYSTPWT (sequence 126) |

**[Table 22]**

| CDR sequence of antibody Ab10-1 | | |
|---|---|---|
| Heavy chain CDR1 | Heavy chain CDR2 | Heavy chain CDR3 |
| GYTFTGYYMH (sequence 55) | WINPNSSGTNYAQKFQG (sequence 127) | PAPRLRGAFDI (sequence 57) |
| Light chain CDR1 | Light chain CDR2 | Light chain CDR3 |
| SGSSSNIGNKVS (sequence 58) | DDNKRPS (sequence 59) | ATWDSSLRAV (sequence 60) |

**[Table 23]**

| CDR sequence of antibody Ab10-2 | | |
|---|---|---|
| Heavy chain CDR1 | Heavy chain CDR2 | Heavy chain CDR3 |
| GYTFTGYYMH (sequence 55) | WINPNSGGTNYAQKFQG (sequence 56) | LAPRLRGAFDI (sequence 128) |
| Light chain CDR1 | Light chain CDR2 | Light chain CDR3 |
| SGSSSNIGNKVS (sequence 58) | DDNKRPS (sequence 59) | ATWDSSLRAV (sequence 60) |

**[Table 24]**

| CDR sequence of antibody Ab10-3 | | |
|---|---|---|
| Heavy chain CDR1 | Heavy chain CDR2 | Heavy chain CDR3 |
| GYTFTGYYMH (sequence 55) | WINPNSGGTNYAQKFQG (sequence 56) | PAWRLRGAFDI (sequence 129) |
| Light chain CDR1 | Light chain CDR2 | Light chain CDR3 |
| SGSSSNIGNKVS (sequence 58) | DDNKRPS (sequence 59) | ATWDSSLRAV (sequence 60) |

**[Table 25]**

| CDR sequence of antibody Ab10-4 | | |
|---|---|---|
| Heavy chain CDR1 | Heavy chain CDR2 | Heavy chain CDR3 |
| GYTFTGYYMH (sequence 55) | WINPNSGGTNYAQKFQG (sequence 56) | PAPRLRMAFDI (sequence 130) |
| Light chain CDR1 | Light chain CDR2 | Light chain CDR3 |
| SGSSSNIGNKVS (sequence 58) | DDNKRPS (sequence 59) | ATWDSSLRAV (sequence 60) |

**[Table 26]**

| CDR sequence of antibody Ab10-5 | | |
|---|---|---|
| Heavy chain CDR1 | Heavy chain CDR2 | Heavy chain CDR3 |
| GYTFTGYYMH (sequence 55) | WINPNSGGTNYAQKFQG (sequence 56) | PAPRLRGAYDI (sequence 131) |
| Light chain CDR1 | Light chain CDR2 | Light chain CDR3 |
| SGSSSNIGNKVS (sequence 58) | DDNKRPS (sequence 59) | ATWDSSLRAV (sequence 60) |

**[Table 27]**

| CDR sequence of antibody Ab10-6 | | |
|---|---|---|
| Heavy chain CDR1 | Heavy chain CDR2 | Heavy chain CDR3 |
| GYTFTGYYMH (sequence 55) | WINPNSGGTNYAQKFQG (sequence 56) | PAPRLRGAFDI (sequence 57) |
| Light chain CDR1 | Light chain CDR2 | Light chain CDR3 |
| SGSSSNIGTKVS (sequence 132) | DDNKRPS (sequence 59) | ATWDSSLRAV (sequence 60) |

**[Table 28]**

| CDR sequence of antibody Ab10-7 | | |
|---|---|---|
| Heavy chain CDR1 | Heavy chain CDR2 | Heavy chain CDR3 |
| GYTFTGYYMH (sequence 55) | WINPNSGGTNYAQKFQG (sequence 56) | PAPRLRGAFDI (sequence 57) |
| Light chain CDR1 | Light chain CDR2 | Light chain CDR3 |
| SGSSSNIGNFKVS (sequence 133) | DDNKRPS (sequence 59) | ATWDSSLRAV (sequence 60) |

**[Table 29]**

| CDR sequence of antibody Ab10-8 | | |
|---|---|---|
| Heavy chain CDR1 | Heavy chain CDR2 | Heavy chain CDR3 |
| GYTFTGYYMH (sequence 55) | WINPNSGGTNYAQKFQG (sequence 56) | PAPRLRGAFDI (sequence 57) |
| Light chain CDR1 | Light chain CDR2 | Light chain CDR3 |
| SGSSSNIGNKVS (sequence 58) | DDNKRPS (sequence 59) | VTWDSSLRAVV (sequence 134) |

**[Table 30]**

| CDR sequence of antibody Ab10-9 | | |
|---|---|---|
| Heavy chain CDR1 | Heavy chain CDR2 | Heavy chain CDR3 |
| GYTFTGYYMH (sequence 55) | WINPISGGTNYAQKFQG (sequence 135) | PAPRLRGAFDI (sequence 57) |
| Light chain CDR1 | Light chain CDR2 | Light chain CDR3 |
| SGSSSNIGNKVS (sequence 58) | DDNKRPS (sequence 59) | ATWDSSLRAV (sequence 60) |

**[Table 31]**

| CDR sequence of antibody Ab10-10 | | |
|---|---|---|
| Heavy chain CDR1 | Heavy chain CDR2 | Heavy chain CDR3 |
| GYTFTGYYMH (sequence 55) | WINPNTGGTNYAQKFQG (sequence 136) | PAPRLRGAFDI (sequence 57) |

| Light chain CDR1 | Light chain CDR2 | Light chain CDR3 |
|---|---|---|
| SGSSSNIGNKVS (sequence 58) | DDNKRPS (sequence 59) | ATWDSSLRAV (sequence 60) |

In addition, the binding molecule or the fragment thereof according to the present invention may have a heavy chain variable region that comprises an amino acid sequence shown below or an amino acid sequence having a sequence homology of 70% or more, 80% or more, or 90% or more, thereto.

**[Table 32]**

| Sequences of heavy chain variable regions of TCTP-specific antibodies according to the present invention | |
|---|---|
| Antib ody ID | Sequence of heavy chain variable region |
| Ab1 | MDWTWRILFLVAAATGAHSQVQLVQSGAEVKKPGASVKVSCKASGYTFTGYYMHWVRQAPGQGL EWMGWINPNSGGTNYAQKFQGWVTMTRDTSISTAYMELSRLRSDDTAVYYCARGITGTTPFDYW GQGTLVTVSS (SEQ ID NO:137) |
| Ab2 | MDTLCYTLLLLTTPSWVLSQVTLKESGPVLVKPTETLTLTCTVSGFSLSNARMGVSWIRQPPGK ALEWLAHIFSNDEKSYSTSLKSRLTISKDTSKSQVVLTMTNMDPVDTATYYCARVLLWFGELYF DYWGQGTLVTVSS (SEQ ID NO:139) |
| Ab3 | MDWTWRILFLVAATGAHSQVQLVQSGAEVKKPGASVKVSCKASGYTFTGYYMHWVRQAPGQGL EWMGWINPNSGGTNYAQKFQGWVTMTRDTSISTAYMELRLRSDDTAVYYCARLGTTGYYYFDYW GQGTLVTVSS (SEQ ID NO:141) |
| Ab4 | MDWTWRILFLVAAATGAHSQVQLVQSGAEVKKPGASVKVSCKASGYTFTSNAMHWVRQAPGQRL EWMGWINAGNGNTKYSQKFQGRVTITRDTSASTAYMELSSLRSEDTAVYYCARGYYDILTGYYT TDAFDIWGQGTMVTVSS (SEQ ID NO:143) |
| Ab5 | MDWTWRILFLVAAATGAHSQVQLVQSGAEVKKPGASVKVSCKASGYIFISYAMHWVRQAPGQRL EWMGWINAGNGYTEYSQKFQGRVTITRDTSALTAYMELRSLRSEDTAVYYCARRGYFDWWAFDY WGQGTLVTVSS (SEQ ID NO:145) |
| Ab6 | MKHLWFFLLLVAAPRWVLSQVQLQESGPGLVKPSGTLSLTCAVSGGSISSSNWWSWVRQPPGKG LEWIGEIDHSGTTTYNPSLKSRVTMSVDQSKNQFSLKLSSVTAADTAVYYCARDGSGSYEGYWG QGTLVTVSS (SEQ ID NO:147) |
| Ab7 | MKHLWFFLLLVAAPRWVLSQVQLQESGPGLVKPSETLSLTCAVSGGSISSSNWWSWVRQPPGKG LEWIGEIYHGVTTYNPSLKSRVTISVDNSKNQFSLKLSSVTAADTAVYYCARDGSGSYEGYWGQ GTLVTVSS (SEQ ID NO:149) |
| Ab8 | MDWTWRILFLVAAATGAHSQVQLVQSGAEVKKPGASVKVSCKASGYTFFSYAMHWVRQAPGQRL EWMGWINAGNGNTKYSQKFQDRVTITRDTSATTAYMELSSLRSEDTAVYYCASWVFDYWGQGTP VTVSS (SEQ ID NO:151) |
| Ab9 | MSVSFLIFLPVLGLPWGVLSQVQLQQSGPGLVKPSQTLSLTCAISGDSVSSNSAAWNWIRQSPS RGLEWLGRTYYRSKWYNDYAVSVKSRITINPDTSKNQFSLQLNSVTPEDTAVYYCAREGITVGR GVMLYFDLWGRGTLVTVSS (SEQ ID NO:153) |
| Ab10 | QVQLVQSGAEVKKPGASVKVSCKASGYTFTGYYMHWVRQAPGQGLEWMGWINPNSGGTNYAQKF QGRVTMTRDTSISTAYMELSRLRSDDTAVYYCARPAPRLRGAFDIWGQGTLVTVSS (SEQ ID NO:155) |
| Ab12 | MDWTWRILFLVAAATGAHSQVQLVQSGAEVKKPGASVKVSCKASGYTFTSHAVHWVRQAPGQRL EWMGWINAGKGNTKYSQKFQGRVTITRDTSATTAYMELSSLRSEDTAVYYCARGRAVAFDPWGQ GTLVTVSS (SEQ ID NO:157) |
| Ab13 | MSVSFLIFLPVLGLPWGVLSQVQLQQSGPGLVKPSQTLSLTCAISGDSVSSNSAAWNWIRQSPS RGLEWLGRTYYRSKWYNDYAVSVKSRITINPDTSKNQFSLQLNSVTPEDTAVYYCARGGAAVFD YWGQGTLVTVSS (SEQ ID NO:159) |
| Ab14 | MDWTWRILFLVAAATGAHSQVQLVQSGAEVKKPGASVKVSCKASGYTFTGYYMHWVRQAPGQGL EWMGWINPNSGDTNYAQKFQGWVTMTRDTSISTAYMELSRLRSDDTAVYYCARGGVLLYFGELS RFDYWGQGTLVTVSS (SEQ ID NO:161) |
| Ab15 | MDWTWRILFLVAAATGAHSQVQLVQSGAEVKKPGASVKLSCKAAGYTFTVNAMHWVRQAPGQGL EWMGWINAGTGKTKYSQKFQGRLTITRDTSASTAYMHLSGLRSEDTAVYYCARGRAVAFDPWGQ GTLVTVSS (SEQ ID NO:163) |
| Ab16 | MSVSFLIFLPVLGLPWGVLSQVQLQQSGPGLVKPSQTLSLTCAISGDSVSSNSAAWNWIRQSPS RGLEWLGRTYYKSKWYNDYAVSVKSRMTINPDTSKNQFSLQLNSVTPEDTAVYYCARGGSAVFD YWGQGTLVTVSS (SEQ ID NO:165) |
| Ab17 | MSVSFLIFLPVLGLPWGVLSQVQLQQSGPGLVKPSQTLSLTCAISGDSVSSNSAAWNWIRQSPS RGLEWLGRTYYKSKWYNDYAVSVKSRMTINPDTSKNQFSLQLNSVTPEDTAVYYCARGGSAVFD YWGQGTLVTVSS (SEQ ID NO:167) |
| Ab18 | MSVSFLIFLPVLGLPWGVLSQVQLQQSGPGLVKPSQTLSLTCAISGDSVSSNSAAWNWIRQSPS RGLEWLGRTYYRSKWYNDYAVSVKSRITINPDTSKNQFSLQLNSVTPEDTAVYYCARGGAAVFD YWGQGTLVTVSS (SEQ ID NO:169) |
| Ab19 | MSVSFLIFLPVLGLPWGVLSQVQLQQSGPGLVKPSQTLSLTCAISGDSVSSSSAAWNWIRQSPS RGLEWLGRTYYRSKWYNDYAESVKSRITINPDTSKNQFSLQLNSVTPEDTAVYYCARRLTIVRG VMRGGMDVWGQGTTVTVSS (SEQ ID NO:171) |
| Ab20 | MSVSFLIFLPVLGLPWGVLSQVQLQQSGPGLVKPSQTLSLTCAISGDSVSSSSAAWNWIRQSPS RGLEWLGRTFYRSKWYNDYAESVKSRITINADTSKNQFSLQLNSVTPEDTAVYYCARRLTIVRG VMRGGMDVWGQGTTVTVSS (SEQ ID NO:173) |
| Ab21 | MSVSFLIFLPVLGLPWGVLSQVQLQQSGPGLVKPSQTLSLTCAISGDSVSSSSAAWNWIRQSPS RGLEWLGRTYYRSKWYNDYAESVKSRITINPDTSKNQFSLQLNSVTPEDTAVYYCARRLTIVRG VMRGGMDVWGQGTTVTVSS (SEQ ID NO:175) |
| Ab22 | MSVSFLIFLPVLGLPWGVLSQVQLQQSGPGLVKPSQTLSLTCAISGDSVSSSSAAWNWIRQSPS RGLEWLGRTYYRSKWYNDYAESVKSRITINPDTSKNQFSLQLNSVTPEDTAVYYCARRLTIVRG VMRGGMDVWGQGTTVTVSS (SEQ ID NO:177) |

The underlined portions in the sequence of the variable region described above correspond, in order, to the heavy chains CDR1, CDR2, and CDR3 of the corresponding antibody.

In addition, the binding molecule or the fragment thereof according to the present invention may have a light chain variable region comprising an amino acid sequence shown below or an amino acid sequence having a sequence homology of 70% or more, 80% or more, or 90% or more, thereto.

**[Table 33]**

| Sequences of light chain variable regions of TCTP-specific antibodies according to the present invention | |
|---|---|
| **Antibod y ID** | **Sequence of light chain variable region** |
| Ab1 | MRVPAQLLGLLLLWFPGARCDIQMTQSPSSLSASVGDRVTITCRASQGIRNDLGWYQQKPGKAP KRLIYAASSLQSGVPSRFSGSGSGTEFTLTISSLQPEDFATYYYLQHNSYPYTFGQGTKLEIK (SEQ ID NO:138) |
| Ab2 | MEAPAQLLFLLLLWLPDTTGEIVMTQSPATLSVFPGERATLSCRASQSVSSNLAWYQQKPGQAP RLLIYGASTRATGIPARFSGSGSGTECTLTISSLQSEDFAVYYCQQYNNWPRTFSQGTKVEIK (SEQ ID NO:140) |
| Ab3 | MLPSQLIGFLLLWVPASRGEIVLTQSPDFQSVTPKEKVTITCRASQSIGSSLHWYQQKPDQSPK LLIKYASQSFSGVPSRFSGSGSGTDFTLTINSLEAEDAATYYCHQSSSLPRTFGQGTKVEIK (SEQ ID NO:142) |
| Ab4 | MRLPAQLLGLLMLWVPGSSGDVVMTQSPLSLPVTLGQPASISCRSSQSLVYSDGNTYLNWFQQR PGQSPRRLIYKVSNRDSGVPDRFSGSGSGTDFTLKITRVEAEDVGVYYCMQGTHWPYTFGQGTK LEIK (SEQ ID NO:144) |
| Ab5 | MLPSQLIGFLLLWVPASRGEIVLTQSPDFQSVTPKEKVTITCRASQSIGSSLHWYQQKPDQSPK LLIKYASQSFSGVPSRFSGSGSGTDFTLTINSLEAEDAATYYCHQSSSLPQTFGQGTKVEIK (SEQ ID NO:146) |
| Ab6 | MRVLAQLLGLLLLCFPGARCDIQMTQSPSSLSASVGDRVTITCRASQAISNHLAWFQQKPGKAP KSLIYAASSLQSGVPSKFSGSGSGTDFTLTISSLQPEDFATYYCQQYHSYPITFGQGTRLEIK (SEQ ID NO:148) |
| Ab7 | MRVLAQLLGLLLLCFPGARCDIQMTQSPSSLSASVGDRVTITCRASQDISNYLAWFQQKPGKAP KSLIYAASSLQSGVPSKFSGSGSGTDFTLTISSLQPEDFATYYCQQYHYYPITFGQGTRLEIK (SEQ ID NO:150) |
| Ab8 | MRLLAQLLGLLMLWVPGSSGDIVMTQTPLSSLVTLGQPASISCRSSQSLVHRDGNTYLSWLQQR PGQPPRLLIYKISNRFFGVPDRFSGSGAGTDFTLKISRVEAEDVGVYYCMQATQFPHTFGQGTK LEIK (SEQ ID NO:152) |
| Ab9 | MRLPAQLLGLLMLWVPGSSGDVLMTQSPLSLPVTLGQPASISCRSSQSLVYSDGNTHLKWFQQR PGQSPRRLIYKVSKWDSGVPDRVSGSGSGTDFTLKISRVEAEDVGVYYCMQGTHWPPTFGQGTK VEIK (SEQ ID NO:154) |
| Ab10 | QSVVTQPPSVSAAPGQKVTISCSGSSSNIGNKKVSWYQQLPVTAPKLLIYDDNKRPSGIPDRFS GSKSGTSATLGITGLQTGDEADYYCATWDSSLRAVVFGGGTKLTVL (SEQ ID NO:156) |
| Ab12 | MAWALLLLTLLTQGTGSWARSALTQPPSASGSPGQSVTVSCTGTNSDIGGYNYVSWYKQHPGNA PKLMIYEVNKRPSGVPDRFSGSKSGNTASLTVSGLQTEDEADYYCASYAGDNNLVFGGGTKLTV L (SEQ ID NO:158) |
| Ab13 | MVLQTQVFISLLLWISGAYGDIVMTQSPDSLAVSLGERATINCKSSQSILYNSNNKNYLAWYQQ NPGQPPKLLFYWASTRGSGVPDRFSGSGSGTDFTLTISSLQAEDVAVYYCQQYYSTPLTFGGGT KVEIK (SEQ ID NO:160) |
| Ab14 | MAWALLLLTLLTQGTGSWAQSALTQPPSASGSPGQSVAISCTGTSSDVGGYNYVSWYQQHPGKA PQLLIYEVTKRPSGVPDRFSGSKSGNTASLTVSGLQAEDEADYYCASYAGDNNLVFGGGTKLTV L (SEQ ID NO:162) |
| Ab15 | MAWALLLLTLLTQGTGSWAQSALTQPPSASGSPGQSVTISCTGTSSDVGGYDYVSWYQQHPDKA PKLMIYEVNKRPSGVPDRFSGSKSGNTASLTVSGLQAEDEADYYCNSYAGNNNLVFGGGTKLTV L (SEQ ID NO:164) |
| Ab16 | MVLQTQVFISLLLWISGAYGDIVMTQSSDSLAVSLGERATINCKSSQSILYSSNNKNYLAWYQQ KLGQPPNLLIYWASTRKSGVPDRFSGSGSGTDFTLTISSLQAEDVAVYYCQQYYGLPLTFGGGT KVEIK (SEQ ID NO:166) |
| Ab17 | MVLQTQVFISLLLWISGAYGDIVMTQSSDSLAVSLGERATINCKSSQSVLYSSNNKNYLAWYQQ KLGQPPNLLIYWASTRKSGVPDRFSGSGSGTDFTLTISSLQAEDVAVYYCQQYYGIPLTFGGGT KVEIK (SEQ ID NO:168) |
| Ab18 | MVLQTQVFISLLLWISGAYGDIVMTQSPDSLAVSLGERATINCKSSQSILYSSNNKNYLAWYQQ NPGQPPKLLFYWASTRGSGVPDRFSGSGSGTDFTLTISSLQAEDVAVYYCQQYYSTPLTFGGGT KVEIK (SEQ ID NO:170) |
| Ab19 | MVLQTQVFISLLLWISGASGDIVMTQSPDSLAVSLDERATINCKSSQNLLYSANNKDYLAWYQQ KPGQPPKLLIYWASTRYSGIPDRFSGSGSGTDFTLTISSLQAEDVAVYYCQQYYSTPWTFGQGT KVEIK (SEQ ID NO:172) |
| Ab20 | MVLQTQVFISLLLWISGASGDIVMTQSPDSLTGSLDERATINCKSSQNLLYSANNKDYIAWYQQ KPGQSPKLLIYWASIRYSGVPDRFSGSGSGTDFTLTISSLQAEDVAVYYCQQYFSTPWTFGQGT KVEIK (SEQ ID NO:174) |
| Ab21 | MVLQTQVFISLLLWISGASGDIVMTQSPDSLAVSLDERATINCKSSQNLLYSANNKDYLAWYQQ KPGQSPKLLIYWASTRYSGVPDRFSGSGSGTDFTLTISSLQAEDVAVYYCQQYYSTPWTFGQGT KVEIK (SEQ ID NO:176) |
| Ab22 | MVLQTQVFISLLLWISGASGDIVMTQSPDSLAVSLDERATINCKSSQNLLYSANNKDYLAWYQQ KPGQSPKLLIYWASTRYSGVPDRFSGSGSGTDFTLTISSLQAEDVAVYYCQQYYSTPWTFGQGT KVEIK (SEQ ID NO:178) |

The underlined portions in the sequence of the variable region described above correspond, in order, to the light chains CDR1, CDR2, and CDR3 of the corresponding antibody.

The sequence homology to polypeptides is also referred to as sequence similarity or sequence identity and is generally measured using sequence analysis software. Protein analysis software matches similar sequences by using the similarity measures assigned to various substitutions, deletions, and other modifications, including conservative amino acid substitutions. For example, GCG software includes programs such as Gap and Bestfit, which can be used together with default parameters for measuring sequence homology or sequence identity between closely related polypeptides, for example, homologous polypeptides from different species of organisms, or between wild-type proteins and mutants thereof (GCG version 6.1). Polypeptide sequences can also be compared by using FASTA that uses default or recommended parameters or by using a GCG version 6.1 program. FASTA (for example, FASTA2 and FASTA3) provides alignment and sequence identity in terms of % for the largest overlapping region between unknown sequences and a search sequence (Pearson 2000). In a case of comparing the sequences according to the present invention with a database containing a large number of sequences from different organisms, another preferred algorithm is BLAST, which is a computer program using default parameters, particularly BLASTP or TBLASTN [see the literature, Altschul et al. (1990) J. Mol. Biol. 215:403-410, and Altschul et al. (1997) Nucleic Acids Res. 25:3389-402].

The binding molecule or the fragment thereof according to the present invention may be a binding molecule or a fragment thereof that competes for the binding to human TCTP with an antibody comprising the heavy chain CDR1 comprising the amino acid sequence set forth in SEQ ID NO:1, 7, 13, 19, 25, 31, 37, 43, 49, 55, 61, 67, 73, 79, 85, 91, 97, 103, 109, 115, 115, or 121, the heavy chain CDR2 comprising the amino acid sequence set forth in SEQ ID NO:2, 8, 14, 20, 26, 32, 38, 44, 50, 56, 62, 68, 74, 80, 86, 92, 98, 104, 110, 116, 122, 127, 135, or 136, the heavy chain CDR3 comprising the amino acid sequence set forth in SEQ ID NO:3, 9, 15, 21, 27, 33, 39, 45, 51, 57, 63, 69, 75, 81, 87, 93, 99, 105, 111, 117, 123, 128, 129, 130, or 131, the light-chain CDR1 comprising the amino acid sequence set forth in SEQ ID NO:4, 10, 16, 22, 28, 34, 40, 46, 52, 58, 64, 70, 76, 82, 88, 94, 100, 106, 112, 118, 124, 132, or 133, the light chain CDR2 comprising the amino acid sequence set forth in SEQ ID NO:5, 11, 17, 23, 29, 35, 41, 47, 53, 59, 65, 71, 77, 83, 89, 95, 101, 107, 113, 119, or 125, and the light chain CDR3 comprising the amino acid sequence set forth in SEQ ID NO:6, 12, 18, 24, 30, 36, 42, 48, 54, 60, 66, 72, 78, 84, 90, 96, 102, 108, 114, 120, 126, or 134.

The phrase "competes for binding" that is used in the present specification means that a binding molecule, particularly an antibody or an antigen-binding fragment thereof, binds to an antigen and inhibits or blocks the binding of another antibody or an antigen-binding fragment thereof to the antigen. This term also covers competition in both directions between two antibodies, that is, a first antibody blocks the binding of a second antibody to an antigen and the first antibody binds to the antigen, and vice versa. In a specific exemplary embodiment, the first antibody and the second antibody can bind to the same epitope. Alternatively, the first and second antibodies can bind to different but overlapping epitopes so that one binding blocks and inhibits the binding of the second antibody, for example, through steric hindrance. Cross-competition between antibodies can be measured by a method known in the related field, for example, by a bio-layer interferometry analysis which is real-time and label-free.

TCTP is also called histamine secretion factor (HRF), Tpt1, p23, or Fortilin. The amino acid sequence and nucleic acid sequence of TCTP can be searched in NCBI through the accession numbers CCDS9397.1 (cDNA) and NP_003286.1 (amino acid). The term "TCTP" as used in the present application is interpreted to include not only the full-length form of TCTP (including variants) but also a cleaved form thereof or a multimer thereof (for example, a dimer). The term "variant" may mean a form in which the polypeptide backbone (for example, amino acid sequence) of TCTP has changed, or a form in which the TCTP polypeptide has undergone a post-translational modification (for example, glycosylation or phosphorylation).

The term "specifically binding" that is used in the present specification refers to a non-specific interaction and a measurably different binding. Specific binding can be determined by competing with a control group molecule similar to a target that does not have binding activity. The assay for "specific binding" can be carried out using a method publicly known in the related field, and examples of such a method include, but are not limited to, Western blotting, ELISA, RIA, ECL, IRMA, a surface plasmon resonance (SPR) test, and peptide scanning.

The term "degree of affinity" or "affinity" or "binding capacity" that is used in the present specification is the strength of interaction between an antibody or an antigen-binding fragment thereof and an antigen, and it is determined by the characteristics of the antigen such as the size, shape, and/or charge of the antigen and the CDR sequence of the antibody or antigen-binding fragment. A method for determining such affinity is publicly known in the related art.

The term "binding molecule" that is used in the present specification refers to any molecule that recognizes TCTP or can specifically bind to TCTP by interacting with TCTP. The binding molecule according to the present invention is preferably a polypeptide and can include a protein moiety and a non-protein moiety (for example, a chemical linker, a crosslinking agent, a drug, and the like). Preferably, the polypeptide is an antibody.

The term "antibody" that is used in the present specification refers to an intact immunoglobulin of any isotype, or an antigen-binding fragment capable of competing with an intact antibody for binding to a target antigen. Examples thereof include a chimeric, humanized, fully human, or bispecific antibody or an antigen-binding fragments thereof. The antibody itself is also a type of antigen-binding protein. In general, the intact antibody contains at least two full-length heavy chains and two full-length light chains. However, in some cases, the antibody may contain only a heavy chain, as naturally found in animals of the Camelidae. The antibody or the antigen-binding fragment thereof may only be derived from a single source or may be a chimera. The chimeric antibody includes a moiety derived from two kinds of different antibodies, which will be described in more detail below. The antibody or the antigen-binding fragment thereof can be produced by a hybridoma (for example, see the literature, Kohler and Milstein 1975), a recombinant DNA technology (for example, US Patent No. 4816567), or enzymatic or chemical cutting of an intact antibody. As an alternative method, it can be isolated with such a method that uses a phage antibody library (for example, Clarkson et al., 1991; Marks et al., 1991). The antibody can also be obtained by carrying out B-cell screening or hybridoma screening by using a mouse obtained by a genetic engineering technique, for example, a humanized mouse (for example, CaMouse^{®}) (https:// www.berkeleylights.com/systems/Beacon/, Rapid single B cell antibody discovery and structured light, mABs, https:// doi. Org/10.1080/19420862.2019.1624126). Unless otherwise stated, the term "antibody" in the present specification includes an antibody containing two full-length heavy chains and two full-length light chains, as well as a derivative thereof, a mutant thereof, a fragment thereof, and a mutant thereof, and examples thereof are described below.

In the present specification, the "light chain" includes a full-length light chain and a fragment thereof, which have a sequence of a variable region sufficient to provide binding specificity for an antigen or an epitope. The full-length light chain comprises a variable region domain VL and a constant region domain CL. The variable region domain of the light chain is present at the amino terminal of the light chain polypeptide. The kinds of light chains include kappa and lambda chains.

In the present specification, the "heavy chain" includes a full-length heavy chain and a fragment thereof, which have a sequence of a variable region sufficient to provide binding specificity for an antigen or an epitope. The full-length heavy chain comprises a variable region domain VH and three constant region domains CH1, CH2, and CH3. The VH domain is present at the amino terminal of the heavy chain polypeptide, the CH domain is present at the carboxy terminal, and CH3 is located closest to the carboxy-terminal. The heavy chain includes isotypes of IgG (including IgG1, IgG2a, IgG2b, IgG3, and IgG4 subtypes), IgA (including IgA1 and IgA2 subtypes), IgM, and IgE.

The term "fragment" as used in the present specification means an "antigen binding fragment," and a binding molecule, particularly an "antigen binding fragment" of an antibody or an immunoglobulin chain (heavy chain or light chain), includes a part of an antibody that can specifically bind to an antigen although lacking some amino acids as compared with the full-length chain, Such a fragment can be said to be biologically active in terms of being able to specifically bind to a target antigen or being able to compete with other antibodies or antigen-binding fragments to bind to a specific epitope. In one aspect, such a fragment comprises at least one CDR that is present within the full-length light chain or heavy chain, and in some exemplary embodiments, includes a short-chained heavy chain and/or light chain, or a part thereof. Such a biologically active fragment can be produced by a recombinant DNA technology or can be produced, for example, by enzymatically or chemically cleaving an intact antibody. Examples of the immunologically functional immunoglobulin fragment include, but are not limited to, Fab, Fab', F(ab')₂, Fv, a domain antibody, and a short chain antibody. In addition, it can be derived from any mammal including a human, a mouse, a rat, a camelid, or a rabbit, although not limited to this. A functional moiety of an antibody, such as one or more CDRs described in the present application can be covalently linked to a second protein or low molecular weight compound, thereby being capable of being used as a targeted therapeutic agent for a specific target.

The "Fab fragment" is composed of one light chain and one heavy chain containing only CH1 and a variable region. The heavy chain of the Fab molecule cannot form a disulfide bond with other heavy chain molecules.

The "Fc" region comprises two molecules of heavy chain fragments containing the CH2 and CH3 domains of the antibody. These two heavy chain fragments are bound to each other by two or more disulfide bonds and the hydrophobic interaction of the CH3 domain.

The "Fab fragment" further comprises a region that is present between the CH1 domain and the CH2 domains of the heavy chain, in the Fab fragment, and an interchain disulfide bond is formed between the two heavy chains of the two molecules of the Fab' fragment, whereby the F(ab')₂ molecule can be formed.

As described above, the "F(ab')₂ fragment" comprises two light chains and two molecules of heavy chains which contain a variable region, the CH1 domain, and a part of a constant region between the CH1 domain and the CH2 domain, and an interchain disulfide bond is formed between the two molecules of heavy chains. As a result, the F(ab')₂ fragment is composed of two Fab' fragments, and the two Fab' fragments are associated with each other by disulfide bonds between them.

The "Fv region" is an antibody that contains variable regions of the heavy chain and the light chain but does not contain the constant region.

The "short chain antibody" is a single polypeptide chain of an antigen-binding region in which variable regions of the heavy chain and the light chain are linked by a flexible linker. For the short chain antibody, for example, US Patent No. 5,260,203 can be referenced.

The "domain antibody" is an immunologically functional immunoglobulin fragment that contains only a variable region of the heavy chain or a variable region of the light chain. In an exemplary embodiment, two or more VH regions are covalently linked by a peptide linker to form a bivalent domain antibody. The two VH regions of such a bivalent domain antibody can target the same or different antigens.

The "bivalent antigen-binding protein" or the "bivalent antibody" comprises two antigen binding sites. The two antigen binding sites included in such a bivalent antibody may have the same antigen specificity or may be bispecific antibodies that bind to different antigens, respectively.

The "multispecific antigen-binding protein" or the "multispecific antibody" targets two or more antigens or epitopes.

The "bispecific" or "bispecific" antigen-binding protein or antibody is a hybrid antigen-binding protein or antibody having two antigen binding sites different from each other. This bispecific antibody is one kind of multispecific antigen-binding protein or multispecific antibody, and it can be produced by various publicly known methods, for example, fusion of hybridomas or linking of Fab' fragments. For example, the following literature can be referenced: Songsivilai and Lachmann, Clin. Exp. Immunol. 1990, 79:315-321; and Kostelny et al. J. Immunol. 1992, 148:1547-1553. Two different epitopes to which two antigen-binding sites of the bispecific antigen-binding protein or antibody bind may be located at the same or different protein targets.

The TCTP-specific binding molecule or the fragment thereof according to the present invention binds to TCTP released from tumor cells and suppresses the function of TCTP outside the cell, preferably in the tumor immune microenvironment, thereby capable of inhibiting the function of the suppressive immune cell in the tumor immune microenvironment. The suppressive immune cell may be preferably a bone marrow-derived suppressor cell (MDSC). The bone marrow-derived suppressor cell (MDSC) may be a polymorphonuclear myeloid-derived suppressor cell (PMN-MDSC) or a monocytic myeloid-derived suppressor cell (M-MDSC).

The TCTP-specific binding molecule or the fragment thereof according to the present invention suppresses the function of TCTP outside the cell, preferably in the tumor immune microenvironment, thereby being capable of activating the function of the anti-cancer immune cell and suppressing tumor proliferation. It can activate or enhance T cells and/or NK cells. The anti-cancer immune cell may be preferably a T cell or an NK cell.

The TCTP-specific binding molecule or the fragment thereof according to the present invention suppresses the function of TCTP outside the cell, preferably in the tumor immune microenvironment, thereby capable of acting as an antagonist against suppressive immune cells in the tumor immune microenvironment and suppressing tumor proliferation. The suppressive immune cell may be preferably a bone marrow-derived suppressor cell (MDSC). The bone marrow-derived suppressor cell (MDSC) may be PMN-MDSC or M-MDSC.

The TCTP-specific binding molecule or the fragment thereof according to the present invention suppresses the function of TCTP outside the cell, preferably in the tumor immune microenvironment, thereby capable of inhibiting the accumulation or recruitment of suppressive immune cells in the tumor immune microenvironment and suppressing tumor proliferation. The suppressive immune cell may be preferably a bone marrow-derived suppressor cell (MDSC). The bone marrow-derived suppressor cell (MDSC) may be PMN-MDSC or M-MDSC.

The TCTP-specific binding molecule or the fragment thereof according to the present invention suppresses the function of TCTP outside the cell, preferably in the tumor immune microenvironment, thereby capable of interfering with binding to an immunoregulatory receptor present on the suppressive immune cell in the tumor immune microenvironment and suppressing tumor proliferation. The suppressive immune cell may be a bone marrow-derived suppressor cell (MDSC), and the immunoregulatory receptor may be Toll-like receptor 2 (TLR2). The bone marrow-derived suppressor cell (MDSC) may be PMN-MDSC or M-MDSC.

The TCTP-specific binding molecule or the fragment thereof according to the present invention interferes with the binding of TCTP to an immunoregulatory receptor present on the suppressive immune cell outside the cell, preferably in the tumor immune microenvironment, thereby capable of inhibiting the activation of the immunoregulatory receptor and inhibiting tumor proliferation. The suppressive immune cell may be preferably a bone marrow-derived suppressor cell (MDSC), and the immunoregulatory receptor may be Toll-like receptor 2 (TLR2). The bone marrow-derived suppressor cell (MDSC) may be PMN-MDSC or M-MDSC.

The TCTP-specific binding molecule or the fragment thereof according to the present invention can be useful for treating or preventing cancer. In this respect, the present invention provides a method for treating or preventing cancer using a TCTP-specific binding molecule or a fragment thereof.

In the present specification, "treatment" means blocking or alleviating the progression and worsening of a disease that occurs in a mammal with cancer. In addition, "prevention" means blocking cancer in advance in a mammalian patient who is at risk of developing cancer.

In addition, the TCTP-specific binding molecule or the fragment thereof according to the present invention can be useful for enhancing responsiveness to a cancer immunotherapeutic agent in an individual being required to be subjected to treatment for cancer, wherein the individual has no response or is likely to have no response to, or has a limited response or is likely to have a limited response to treatment using a cancer immunotherapeutic agent. In this respect, the present invention provides a method for improving responsiveness to a cancer immunotherapeutic agent using a TCTP-specific binding molecule or a fragment thereof.

The term "cancer immunotherapeutic agent" that is used in the present invention collectively refers to medical agents that are used to treat a patient suffering from cancer or at risk of cancer recurrence through a method including inducing, enhancing, suppressing, or modifying an immune response, and the cancer immunotherapeutic agent can have a mechanism for restoring or enhancing the immune system's ability to recognize or destroy tumors in order to overcome the mechanism of immune suppression or immune evasion acquired by cancer cells.

The immune checkpoint inhibitor is one example of such cancer immunotherapeutic agents. The term "immune checkpoint inhibitor" is a medical agent that attacks cancer cells by blocking the activity of immune checkpoint proteins involved in suppressing immune cells, thereby activating immune cells. As the immune checkpoint protein that can be targeted by the immune checkpoint inhibitor, CTLA-4, PD-1, PD-L1, TIM3, LAG3, BTLA, TIGIT, VISTA, GITR, CD47, KIR, and the like are known.

In addition, the TCTP-specific binding molecule or the fragment thereof according to the present invention can also be useful for removing, alleviating, or preventing immune suppression in a tumor microenvironment of a cancer patient. In this respect, the present invention provides a method for eliminating, alleviating, or preventing immune suppression using a TCTP-specific binding molecule or a fragment thereof.

In addition, the TCTP-specific binding molecule or the fragment thereof according to the present invention can also be useful for suppressing suppressive immune cells in a tumor microenvironment of a cancer patient. In this respect, the present invention provides a method for suppressing suppressive immune cells using a TCTP-specific binding molecule or a fragment thereof. The suppressive immune cell may be preferably a bone marrow-derived suppressor cell (MDSC). The bone marrow-derived suppressor cell (MDSC) may be PMN-MDSC or M-MDSC.

In the present invention, examples of the cancer include, but are not limited to, hepatocellular carcinoma, cholangiocarcinoma, renal cell carcinoma, squamous cell carcinoma, basal cell carcinoma, metastatic cell carcinoma, adenocarcinoma, gastrinoma, melanoma, fibrosarcoma, myxosarcoma, liposarcoma, leiomyosarcoma, rhabdomyosarcoma, teratoma, angiosarcoma, Kaposi sarcoma, osteosarcoma, chondrosarcoma, lymphangiosarcoma, meningioma, non-Hodgkin lymphoma, Hodgkin's lymphoma, leukemia, brain tumor, head and neck cancer, an epithelial cell-induced neoplasm (epithelial cell carcinoma), lip cancer, oral cancer, esophageal cancer, gastrointestinal cancer, for example, small intestine cancer and stomach cancer, colon cancer, colorectal cancer, liver cancer, bladder cancer, pancreatic cancer, ovarian cancer, cervical cancer, endometrial cancer, lung cancer, breast cancer, thyroid cancer, skin cancer, for example, squamous cell carcinoma and basal cell carcinoma, and prostate cancer. In addition, examples of the cancer also include other known cancers that affect blood cells. The cancer is preferably colorectal cancer, melanoma, fibrosarcoma, pancreatic cancer, liver cancer, or skin cancer.

Methods that use the TCTP-specific binding molecule or the fragment thereof according to the present invention may comprise a step of administering a therapeutically effective amount of a TCTP-specific binding molecule or a fragment thereof to an individual. The "mammal" subjected to prevention or treatment in the present invention refers to any animal classified as a mammal, and it is for example, in addition to a human, pets such as a dog, a cat, a rabbit, and a ferret, and livestock animals such as a cow, a pig, a sheep, and a horse, which are not specifically limited. A particularly preferred "mammal" is a human.

The present invention also provides a pharmaceutical composition containing a TCTP-specific binding molecule or a fragment thereof as an active ingredient for therapeutic or prophylactic use.

The composition may comprise pharmaceutically acceptable inactive ingredients [see Handbook of Pharmaceutical Excipients, and the like]. The compositions can be produced by being mixed with a physiologically acceptable carrier, excipient, or stabilizer, for example, in a form of a freeze-dried powder, a slurry, an aqueous solution, or a suspension.

The pharmaceutically acceptable carrier contained in the pharmaceutical composition according to the present invention is such one that is commonly used in the formulation and examples thereof include, which are not limited to, lactose, dextrose, sucrose, sorbitol, mannitol, starch, gum acacia, calcium phosphate, alginate, gelatin, calcium silicate, microcrystalline cellulose, polyvinyl pyrrolidone, cellulose, water, syrup, methyl cellulose, methyl hydroxybenzoate, propyl hydroxybenzoate, talc, magnesium stearate, and mineral oil.

In addition to the above components, the pharmaceutical composition according to the present invention may further contain a lubricant, a wetting agent, a sweetening agent, a flavoring agent, an emulsifier, a suspending agent, a preservative, and the like. The pharmaceutically acceptable carriers and pharmaceutical preparations which can be used conventionally are described in detail in Remington's Pharmaceutical Sciences (19th ed., 1995).

The pharmaceutical composition according to the present invention can be administered orally or parenterally, and it can be administered in such a manner as intravenous injection, subcutaneous injection, intradermal injection, intramuscular injection, intraperitoneal injection, intrasternal injection, intratumoral injection, topical administration, intranasal administration, intracerebral administration, intracranial administration, intrapulmonary administration, or rectal administration, which is not limited thereto.

The suitable administration dosage of the pharmaceutical composition according to the present invention can be adjusted according to various factors including the kind of disease, the severity of the disease, the kinds and contents of the active ingredient and other ingredients contained in the pharmaceutical composition, and the kind of formulation, the administration method, as well as the age, body weight, gender, and diet of the patient, the administration time, the administration route, the treatment period, and drugs to be used simultaneously, where usually, an experienced doctor can easily determine and prescribe an effective administration dosage for the desired treatment. For example, the daily administration dosage of the pharmaceutical composition according to the present invention may be 0.001 to 1,000 mg (in terms of active ingredient weight), and continuous administration or intermittent administration is preferred. In the present specification, the term "therapeutically effective amount" means an amount sufficient for treating a disease or condition according to the context.

The pharmaceutical composition according to the present invention may be produced in a unit dose form or produced to be contained in a multi-dosage container by formulating it using a pharmaceutically acceptable carrier and/or excipient according to a method that can be easily carried out by those skilled in the art. In this case, the formulation form may be a form of a solution, suspension, or emulsion in an oily or aqueous medium or may be a form of an extract, a powdered drug, a suppository, a powder, a granule, a tablet, or a capsule, and a dispersant or stabilizer may be further contained.

The pharmaceutical composition according to the present invention may comprise additional therapeutically active ingredients in addition to the TCTP-specific binding molecule or the fragment thereof. The additional therapeutically active ingredient may be, for example, another anticancer agent or an immune checkpoint inhibitor, which may be, for example, an anti-PD-1 antibody or an anti-CTLA-4 antibody.

The present invention also provides an isolated polynucleotide encoding the TCTP-specific binding molecule or the fragment thereof according to the present invention, a vector containing the polynucleotide, and an isolated cell containing the polynucleotide or vector. The present invention also provides a method for producing the TCTP-specific binding molecule or the fragment thereof according to the present invention, which comprises a step of culturing the isolated cells to express the TCTP-specific binding molecule or the fragment thereof according to the present invention and then isolating the binding molecule or the fragment, which has been expressed from the cell. Such a method is widely known to those skilled in the field of biotechnology.

Based on the content described above, the present invention may relate to the following (1) to (21). However, the present invention is not limited thereto.
(1) A binding molecule or a fragment thereof, which suppresses a function of a suppressive immune cell and/or activates an anti-cancer immune cell by binding to a human translationally controlled tumor protein (TCTP), a cleaved form thereof, or a multimer thereof with a dissociation constant (Kd) of about 1 × 10⁻⁷ M or less in a case of being measured by surface plasmon resonance, and then suppressing a function thereof outside the cell.
(2) A binding molecule or a fragment thereof, which specifically binds to TCTP, a post-translational variant thereof, a cleaved form thereof, or a multimer thereof, which comprises heavy chain CDR1, heavy chain CDR2, heavy chain CDR3, light chain CDR1, light chain CDR2, and light chain CDR3 which are respectively as defined in the following (a) to (f), in which
   (a) the heavy chain CDR1 comprises
      (a1) an amino acid sequence of X1-X2-X3-X4-H (where X1 is S, G, or V, X2 is Y, H, or N, X3 is A or Y, and X4 is M or V),
      (a2) an amino acid sequence of S-X5-X6-X7-X8-W-X9 (where X5 is S or N, X6 is N or S, X7 is A or W, X8 is A or is not present, and X9 is N or S),
      (a3) an amino acid sequence of G-Y-T-F-T-G-Y-M-H, or
      (a4) an amino acid sequence of N-A-R-M-G-V-S, or
      a conservative amino acid substituent thereof;
   (b) the heavy chain CDR2 comprises
      (b1) an amino acid sequence of E-I-X1-H-X2-G-X3-T-Y-N-P-S-L-K-S (where X1 is D or Y, X2 is S or is not present, and X3 is T or V),
      (b2) an amino acid sequence of W-I-N-X4-X5-X6-X7-X8-T-X9-Y-X10-Q-K-F-Q-X11 (where X4 is A or P, X5 is G, I, or N, X6 is K, N, S, or T, X7 is G or S, X8 is G, N, Y, D, or K, X9 is K, N, or E, X10 is A or S, and X11 is D or G),
      (b3) an amino acid sequence of R-T-X12-Y-X13-S-W-Y-N-D-Y-A-X14-S-V-K-S (where X12 is F or Y, X13 is K or R, and X14 is E or V), or
      (b4) an amino acid sequence of H-I-F-S-N-D-E-K-S-Y-S-Y-S-L-K-S, or
      a conservative amino acid substituent thereof;
   (c) the heavy chain CDR3 comprises
      (c1) an amino acid sequence of G-X1-X2-X3-X4-F-D-X5 (where X1 is R or G, X2 is A or S, X3 is A or V, X4 is A or V, and X5 is P or Y),
      (c2) an amino acid sequence of D-G-S-G-S-Y-E-G-Y,
      (c3) an amino acid sequence of W-V-F-D-Y,
      (c4) an amino acid sequence of X6-A-X7-R-L-X8-AX9-D-I (where X6 is L or P, X7 is P or W, X8 is G or M, and X9 is F or Y),
      (c5) an amino acid sequence of R-L-T-I-V-R-G-V-M-R-G-M-D-V,
      (c6) an amino acid sequence of G-Y-Y-D-I-L-T-G-Y-T-T-D-A-F-D-I,
      (c7) an amino acid sequence of X10-X11-X12-X13-X14-X15-X16-X17-X18-F-D-Y (where X10 is V or is not present, X11 is R or L or is not present, X12 is G or L, X13 is Y, I, T, or W, X14 is F or T, X15 is D or G, X16 is W, T, Y, or E, X17 is W, T, Y, or L, and X18 is A, P, or Y),
      (c8) an amino acid sequence of G-G-V-L-Y-F-G-E-L-S-R-F-D-Y, or
      (c9) an amino acid sequence of E-G-I-T-V-G-R-G-M-L-Y-F-D-L, or
      a conservative amino acid substituent thereof;
   (d) the light chain CDR1 comprises
      (d1) an amino acid sequence of R-A-S-Q-X1-X2-X3-X4-X5-L-X6 (where X1 is A, D, or G, X2 is I or V, X3 is S, R, or G, X4 is N or S, X5 is H, Y, D, N, or S, and X6 is A, G, or H),
      (d2) an amino acid sequence of X7-G-X8-X9-S-X10-X11-G-X12-X13-X14-X15-V-S (where X7 is S or T, X8 is S or T, X9 is N or S, X10 is D or N, X11 is I or V, X12 is G, N, or T, X13 is F, K, or Y, X14 is N or is not present, and X15 is K or Y),
      (d3) an amino acid sequence of K-S-S-Q-X16-X17-L-Y-X18-X19-N-N-K-X20-Y-X21-A (where X16 is N or S, X17 is I, L, or V, X18 is N or S, X19 is A or S, X20 is D or N, and X21 is I or L), or
      (d4) an amino acid sequence of R-S-S-Q-S-V-X22-X23-D-G-N-T-X24-L-X25 (where X22 is H or Y, X23 is R or S, X24 is Y or H, and X25 is S, N, or K), or
      a conservative amino acid substituent thereof;
   (e) the light chain CDR2 comprises an amino acid sequence of X1-X2-X3-X4-X5-X6-X7 (where X1 is A, D, E, G, K, W, or Y, X2 is A, D, I, or V, X3 is N, S, or T, X4 is I, K, N, Q, S, or T, X5 is L, R, S, or W, X6 is A, D, F, G, K, P, Q, or Y, and X7 is F, S or T) or a conservative amino acid substituent thereof; and
   (f) the light chain CDR3 comprises
      (f1) an amino acid sequence of X1-Q-X2-X3-X4-X5-P-X6-T (where X1 is M or Q, X2 is A, G, or Y, X3 is F, H, N, S, or Y, X4 is G, H, Q, N, S, or Y, X5 is F, I, L, T, Y, or W, and X6 is H, I, L, P, R, W, or Y),
      (f2) an amino acid sequence of X7-X8-X9-X10-X11-X12-X13-X14-X15-V (where X7 is A, N, or V, X8 is S or T, X9 is W or Y, X10 is A or D, X11 is G or S, X12 is D, N, or S, X13 is L or N, X14 is N or R, and X15 is A or L), or
      (f3) an amino acid sequence of X16-Q-X17-X18-S-X19-P-X20-T (where X16 is L or H, X17 is H or S, X18 is N or S, X19 is Y or L, and X20 is Y, R, or Q), or
      a conservative amino acid substituent thereof.
(3) The binding molecule or the fragment thereof according to (1) or (2), in which the heavy chain CDR1 comprises the amino acid sequence set forth in SEQ ID NO:1, 7, 13, 19, 25, 31, 37, 43, 49, 55, 61, 67, 73, 79, 85, 91, 97, 103, 109, 115, 115, or 121, or a conservative amino acid substituent thereof, the heavy chain CDR2 comprises the amino acid sequence set forth in SEQ ID NO:2, 8, 14, 20, 26, 32, 38, 44, 50, 56, 62, 68, 80, 76, 74, 74, 86, 92, 98, 104, 110, 116, 122, 127, 135, or 136, or a conservative amino acid substituent thereof, the heavy chain CDR3 comprises the amino acid sequence set forth in SEQ ID NO:3, 9, 15, 21, 27, 33, 39, 45, 51, 57, 63, 69, 75, 81, 87, 93, 99, 105, 111, 117, 123, 128, 129, 130, or 131, or a conservative amino acid substituent thereof, the light-chain CDR1 comprises the amino acid sequence set forth in SEQ ID NO:4, 22, 16, 10, 16, 28, 34, 40, 46, 52, 58, 64, 70, 76, 82, 88, 94, 100, 106, 112, 118, 124, 132, or 133, or a conservative amino acid substituent thereof, the light chain CDR2 comprises the amino acid sequence set forth in SEQ ID NO:5, 11, 17, 23, 29, 35, 41, 47, 53, 59, 65, 71, 77, 83, 89, 95, 101, 107, 113, 119, or 125, or a conservative amino acid substituent thereof, and the light chain CDR3 comprises the amino acid sequence set forth in SEQ ID NO:6, 12, 18, 24, 30, 36, 42, 48, 54, 60, 66, 72, 78, 84, 90, 96, 102, 108, 114, 120, 126, or 134,
   or a conservative amino acid substituent thereof.
(4) The binding molecule or the fragment thereof according to any one of (1) to (3), in which the heavy chain variable region comprises the amino acid sequence selected from the group consisting of SEQ ID NOs:137, 139, 141, 143, 145, 147, 149, 151, 153, 155, 157, 159, 161, 163, 165, 167, 169, 171, 173, 175, and 177, or the amino acid sequence that has a sequence homology of at least 90% thereto, and the light chain variable region comprises the amino acid sequence selected from the group consisting of SEQ ID NOs:138, 140, 142, 144, 146, 148, 150, 152, 154, 156, 158, 160, 162, 164, 166, 168, 170, 172, 174, 176, and 178or the amino acid sequence that has a sequence homology of at least 90% thereto.
(5) The binding molecule or the fragment thereof according to any one of (1) to (4),
   in which the binding molecule or the fragment thereof competes for the binding to human TCTP with the antibody comprising the heavy chain CDR1 comprising the amino acid sequence set forth in SEQ ID NO:1, 7, 13, 19, 25, 31, 37, 43, 49, 55, 61, 67, 73, 79, 85, 91, 97, 103, 109, 115, 115, or 121, the heavy chain CDR2 comprising the amino acid sequence set forth in SEQ ID NO:2, 8, 14, 20, 26, 32, 38, 44, 50, 56, 62, 68, 74, 80, 86, 92, 98, 104, 110, 116, 122, 127, 135, or 136, the heavy chain CDR3 comprising the amino acid sequence set forth in SEQ ID NO:3, 9, 15, 21, 27, 33, 39, 45, 51, 57, 63, 69, 75, 81, 87, 93, 99, 105, 111, 117, 123, 128, 129, 130, or 131, the light chain CDR1 comprising the amino acid sequence set forth in SEQ ID NO:4, 10, 16, 22, 28, 34, 40, 46, 52, 58, 64, 70, 76, 82, 88, 94, 100, 106, 112, 118, 124, 132, or 133, the light chain CDR2 comprising the amino acid sequence set forth in SEQ ID NO:5, 11, 17, 23, 29, 35, 41, 47, 53, 59, 65, 71, 77, 83, 89, 95, 101, 107, 113, 119, or 125, and the light chain CDR3 comprising the amino acid sequence set forth in SEQ ID NO:6, 12, 18, 24, 30, 36, 42, 48, 54, 60, 66, 72, 78, 84, 90, 96, 102, 108, 114, 120, 126, or 134.
(6) The binding molecule or the fragment thereof according to any one of (1) to (5),
   in which the binding molecule or the fragment thereof is an antibody or an antigen-binding fragment thereof, comprising the heavy chain CDR1 comprising the amino acid sequence set forth in SEQ ID NO:43, the heavy chain CDR2 comprising the amino acid sequence set forth in SEQ ID NO:44, the heavy chain CDR3 comprising the amino acid sequence set forth in SEQ ID NO:45, the light chain CDR1 comprising the amino acid sequence set forth in SEQ ID NO:46, the light chain CDR2 comprising the amino acid sequence set forth in SEQ ID NO:47, and the light chain CDR3 comprising the amino acid sequence set forth in SEQ ID NO: 48.
(7) The binding molecule or the fragment thereof according to any one of (1) to (5),
   in which the binding molecule or the fragment thereof is an antibody or an antigen-binding fragment thereof, comprising the heavy chain CDR1 comprising the amino acid sequence set forth in SEQ ID NO:55, the heavy chain CDR2 comprising the amino acid sequence set forth in SEQ ID NO:56, the heavy chain CDR3 comprising the amino acid sequence set forth in SEQ ID NO:57, the light chain CDR1 comprising the amino acid sequence set forth in SEQ ID NO:58, the light chain CDR2 comprising the amino acid sequence set forth in SEQ ID NO:59, and the light chain CDR3 comprising the amino acid sequence set forth in SEQ ID NO:60.
(8) The binding molecule or the fragment thereof according to any one of (1) to (5),
   in which the binding molecule or the fragment thereof is an antibody or an antigen-binding fragment thereof, comprising the heavy chain CDR1 comprising the amino acid sequence set forth in SEQ ID NO:61, the heavy chain CDR2 comprising the amino acid sequence set forth in SEQ ID NO:62, the heavy chain CDR3 comprising the amino acid sequence set forth in SEQ ID NO:63, the light chain CDR1 comprising the amino acid sequence set forth in SEQ ID NO:64, the light chain CDR2 comprising the amino acid sequence set forth in SEQ ID NO:65, and the light chain CDR3 comprising the amino acid sequence set forth in SEQ ID NO:66.
(9) The binding molecule or the fragment thereof according to any one of (1) to (5),
   in which the binding molecule or the fragment thereof is an antibody or an antigen-binding fragment thereof, comprising the heavy chain CDR1 comprising the amino acid sequence set forth in SEQ ID NO:109, the heavy chain CDR2 comprising the amino acid sequence set forth in SEQ ID NO:110, the heavy chain CDR3 comprising the amino acid sequence set forth in SEQ ID NO:111, the light chain CDR1 comprising the amino acid sequence set forth in SEQ ID NO:number 112, the light chain CDR2 comprising the amino acid sequence set forth in SEQ ID NO:113, and the light chain CDR3 comprising the amino acid sequence set forth in SEQ ID NO:114.
(10) The binding molecule or the fragment thereof according to any one of (1) to (9),
   in which the binding molecule is an antibody, and the fragment thereof is an antigen-binding fragment.
(11) An isolated polynucleotide that encodes the binding molecule or the fragment thereof according to any one of (1) to (9).
(12) A method of producing the binding molecule or the fragment thereof according to any one of (1) to (9), the method comprising the steps of
   expressing, in a host cell, an isolated polynucleotide that encodes the binding molecule or the fragment thereof according to any one of claim 1 to clam 9 (preferably claim 2) and isolating the expressed binding molecule or fragment thereof.
(13) The binding molecule or the fragment thereof according to any one of (1) to (9) for use in treating or preventing cancer; a method for treating or preventing cancer, including administering the binding molecule or the fragment thereof to an individual required to be subjected to treatment or prevention of cancer; or a pharmaceutical composition for treating or preventing cancer, which contains the binding molecule or the fragment thereof.
(14) The binding molecule or the fragment thereof according to any one of (1) to (9) for enhancing responsiveness to a cancer immunotherapeutic agent in an individual being required to be subjected to treatment for cancer, wherein the individual has no response or is likely to have no response to, or has a limited response or is likely to have a limited response to treatment using a cancer immunotherapeutic agent; a method for promoting responsiveness to a cancer immunotherapeutic agent, comprising administrating the binding molecule or the fragment thereof to an individual who had no response or is likely to have no response to, or had a limited response or is likely to have a limited response to treatment using a cancer immunotherapeutic agent; or a pharmaceutical composition for enhancing responsiveness to a cancer immunotherapeutic agent comprising the binding molecule or the fragment thereof.
(15) The binding molecule or the fragment thereof according to any one of (1) to (9) for use in removing, alleviating, or preventing immune suppression in a tumor microenvironment of cancer patients; a method for eliminating, alleviating, or preventing immune suppression, including administering the binding molecule or the fragment thereof to a cancer patient required to be subjected to removal, alleviation, or prevention of immune suppression in a tumor microenvironment; or a pharmaceutical composition for removing, alleviating, or preventing immune suppression, which contains the binding molecule or the fragment thereof.
(16) The binding molecule or the fragment thereof according to any one of (1) to (9) for use in suppression of suppressive immune cells in a tumor microenvironment of a cancer patient; a method for suppressing suppressive immune cells in a tumor microenvironment, including administering the binding molecule or the fragment thereof to a cancer patient; or a pharmaceutical composition for suppressing suppressive immune cells in a tumor microenvironment, which contains the binding molecule or the fragment thereof.
(17) The binding molecule or the fragment thereof, the method, or the composition according to (16), in which the suppressive immune cell is a bone marrow-derived suppressor cell (MDSC).
(18) The binding molecule or the fragment thereof, the method, or the composition according to any one of (13) to (17), in which the cancer is selected from the group consisting of colorectal cancer, melanoma, fibrosarcoma, pancreatic cancer, liver cancer, and skin cancer.
(19) The method or the composition according to (13) to (17), in which an immune checkpoint inhibitor is used together.

Hereinafter, the present invention will be described in detail according to examples below. However, the following examples are only for the exemplary description of the present invention, and thus the present invention is not limited thereto.

### Example 1: Production of antibodies against TCTP

### Example 1-1: Humanized mouse monoclonal antibody

### Immunization

In order to make an antibody that binds to TCTP, a humanized mouse (CAMouse^{®}) producing human antibodies was immunized. Human TCTP having the following amino acid sequence was used as an antigen.

### Human TCTP:

MIIYRDLISHDEMFSDIYKIREIADGLCLEVEGKMVSRTEGNIDDSLIG
GNASAEGPEGEGTESTVITGVDIVMNHHLQETSFTKEAYKKYIKDYMKSIKGKLEEQRPER
VKPFMTGAAEQIKHILANFKNYQFFIGENMNPDGMVALLDYREDGVTPYMIFFKDGLEMEK
C

50 µg of human TCTP labeled with MYC/DDK on the C-terminal was injected subcutaneously into a male humanized mouse, and then boosted by subcutaneously injecting 25 µg of human TCTP six times at intervals of 2 weeks. Ater 1 week of boosting, blood was collected, and immunization titration (immunization titration) was carried out using a standard ELISA method in which binding to an administered antigen was assayed.

### Hybridoma

According to a standardized protocol, a hybridoma producing a monoclonal antibody against human TCTP was obtained by fusion of SP2/0 mouse myeloma cells and spleen cells isolated from a mouse immunized with human TCTP. After fusion, the fusion with mouse B cells producing antibodies was checked using an ELISA method for the antigen administered to the mouse by using a cell culture medium. Next, hybridomas that produce monoclonal antibodies were selected by carrying out singlecell cloning using the obtained hybridomas. Then, antibodies were produced and purified from hybridomas, and antibodies against human TCTP were selected using a standard ELISA method for assaying binding to TCTP. As a result, antibodies Ab12, Ab13, Ab14, Ab15, Ab16, Ab17, Ab18, Ab19, Ab20, Ab21, Ab22 were obtained, and the sequences thereof were analyzed. The sequences of these antibodies are as disclosed in the present specification.

### Beacon screening

In addition to the hybridoma method, a beacon^{®} screening method (Beacon^{®} Optofluidic System) was used as a method for selecting antibodies against TCTP from the mouse immunized with human TCTP. Isolated mouse spleen cells were selected using beacon screening to select spleen cells that produce antibodies against TCTP, and then the sequences of the variable regions of the selected antibodies were analyzed. The human IgG1 constant region was added to the analyzed sequence of the variable region to produce and purify antibodies. Antibodies that bind to human TCTP were selected using an ELISA method for assaying binding to TCTP. As a result, antibodies Ab1, Ab2, Ab3, Ab4, Ab5, Ab6, Ab7, Ab8, and Ab9 were obtained, and the sequences thereof were analyzed. The sequences of these antibodies are as disclosed in the specification of the present application.

Antibodies Ab1, Ab2, Ab3, Ab4, Ab5, Ab6, Ab7, Ab8, Ab9, Ab12, Ab13, Ab14, Ab15, Ab16, Ab17, Ab18, Ab19, Ab20, Ab21, and Ab22 were obtained from CAmouse^{®} in cooperation with CAMAB (China) and GenScript (China).

### Example 1-2: Phage library screening

In addition to the immunization method, single-chain antibodies against human TCTP were selected from a phage library using a biopanning technique. A human-derived single-chain antibody library (Wuxi Biologics) which is diverse was used. A total of four times of biopanning was carried out to select single-chain antibodies that bind to TCTP from the phage library. Specifically, human TCTP protein at a concentration of 10 µg/ml was adsorbed on the surface of an immune test tube, and then a heavy serum albumin (BSA) buffer solution was added to the test tube to protect the surface on which TCTP was not adsorbed. The phage library was put in an immune test tube and allowed to react at ordinary temperature, and then phages that bound to TCTP were extracted. The extracted phages were used for the next round panning. This process was repeated for a total of four rounds to amplify antigen-specific phages. Polyclonal phages extracted in each round were subjected to the checking of the binding to the antigen through ELISA.

In order to select monoclonal phages that specifically bind to human TCTP from the phage pool obtained through the panning, single colonies were obtained from the phage pool. After producing monoclonal phages from the single colonies obtained, phages that bind to human TCTP were selected using an ELISA method for assaying binding to human TCTP. After removing overlapping clones by analyzing the sequences of selected phages, antibodies were produced and purified by adding a human IgG constant region. Antibodies that bind to human TCTP were selected using an ELISA method for assaying binding to TCTP. As a result, an antibody Ab10 was obtained, and the sequence was analyzed. The sequence of this antibody is as disclosed in the specification of the present application.

### Example 2: Analysis of binding capacity for TCTP

### Example 2-1: ELISA analysis using human TCTP antibody

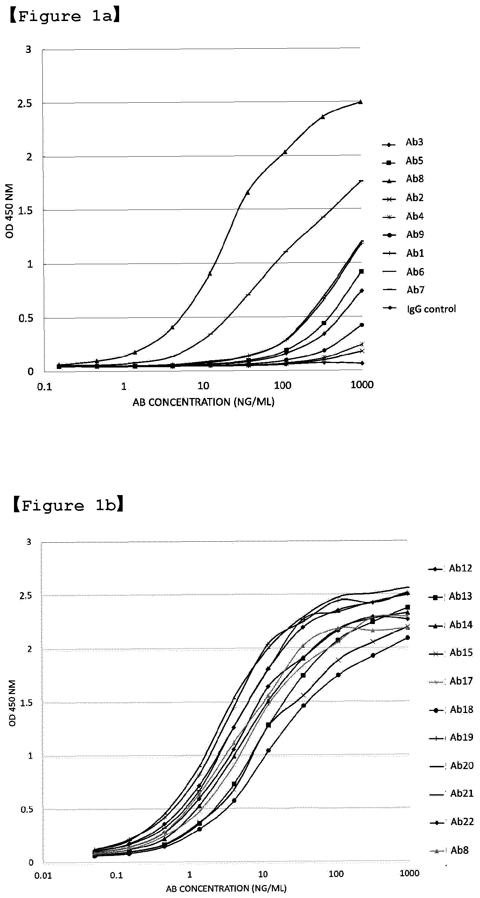

ELISA was carried out to quantitatively analyze the binding capacity of selected antibodies to antigens. To this end, human TCTP was attached to an ELISA plate at a concentration of 1 ug/ml and then treated with antibodies of various concentrations to check the relative binding capacity. As a result of the measurement, all of antibodies Ab1, Ab2, Ab3, Ab4, Ab5, Ab6, Ab7, Ab8, Ab9, Ab12, Ab13, Ab14, Ab15, Ab17, Ab18, Ab19, Ab20, Ab21, and Ab22 were confirmed to bind well to human TCTP (Figs. 1a and 1b).

### Example 2-2: SPR analysis using human TCTP antibody

The antigen binding capacity of antibodies selected in Example 1 was analyzed using a SPR technique. The device used was Biacore 8K (Ge Healthcare). Human TCTP was immobilized on a chip, and antibodies were shed at 6 or 7 different concentrations. A CM5 chip (GE Healthcare, Cat.29-1275-56), a regeneration buffer (10 mM glycine-HCl (GE Healthcare)), and a driving buffer HBS-EP+, and the like were used. For kinetic analysis, the binding time was set to 100 or 120 seconds, the flow rate was set to 30 or 50 µl/min in the binding phase, and in the dissociation phase, the dissociation time was set to 300 or 360 seconds, and the flow rate was set to 30 or 50 µl/min. The fitting was carried out using a 1:1 combined model, and BIACore Evaluation Software (GE healthcare) was used as the evaluation software.

**[Table 34]**

| Analysis results of SPR binding affinity | | |
|---|---|---|
| Antibody | Binding affinity Kd (M) for human TCTP | Concentration used for measurement (nM) |
| Ab 6 | 7.28E-08 | 800, 400,200,100,50,25,12.5 |
| Ab7 | 1.07E-07 | 1600, 800, 400,200,100,50,25 |
| Ab8 | 1.40E-08 | 400,200,100,50,25,12.5,6.25 |
| Ab10 | 2.24E-10 | 0.078, 0.156, 0.313, 0.625, 1.25, 2.5 |
| Ab12 | 1.68E-10 | 2.5, 1.25, 0.625, 0.3125, 0.15625, 0.078125, 0.0390625 |
| Ab17 | 1.23E-09 | 50, 25, 12.5, 6.25, 3.125, 1.5625, 0.78125 |
| Ab19 | 2.77E-09 | 100, 50, 25, 12.5, 6.25, 3.125, 1.5625 |
| Ab20 | 7.04E-10 | 50, 25, 12.5, 6.25, 3.125, 1.5625, 0.78125 |
| 55F3 | 2.63E-07 | 50, 100, 200, 400, 800, 1600, 3200 |

### Example 3: Development of mutant antibody to improve binding capacity of TCTP antibody and prevent post-translational modification

### Example 3-1: Checking of difference in binding capacity of TCTP antibody to human TCTP and mouse TCTP

As a result of carrying out ELISA using the same method as in Example 2-1 to check the difference in binding capacity of selected antibodies to human TCTP and mouse TCTP, it was confirmed that the binding capacity of the antibody Ab10 to mouse TCTP is low.

The sequences of human TCTP and mouse TCTP have seven amino acids different from each other. Therefore, the binding capacity of ab10 mutants was checked after obtaining a mutation in which the sequence of the mouse TCTP was replaced with the sequence of the human TCTP. As a result, it was confirmed that the binding capacity to mouse TCTP is restored in the mutation in which the 13th leucine in mouse TCTP was replaced with methionine which is the same residue as in human TCTP.

### Example 3-2: Post-translational modification of TCTP antibody (Ab10)

After storing the selected antibody Ab10 at 40°C for 14 days, an imaging capillary isoelectric focusing (iCEF) test was carried out, and as a result, it was confirmed that the peak ratio of the acidic variant increases from 35.3% to 64.8%. This was estimated to be due to the post-translational modification of the antibody Ab10, and it was confirmed that a post-translational modification is likely to occur in asparagine and serine, which are the 5th and 6th amino acids of the heavy chain CDR2 of the antibody Ab10.

### Example 3-3: Development of mutant antibody to enhance binding capacity of TCTP antibody (Ab10) and prevent post-translational modification

In order to improve the binding capacity of the antibody Ab10 to mouse TCTP, mutants in which one amino acid in the CDR sequence was mutated were produced using a phage library as follows.

**[Table 35]**

| CDR sequences of antibodies Ab10-1 to 10-8 | | | | |
|---|---|---|---|---|
| Antibody ID | Heavy chain CDR2 | Heavy chain CDR3 | Light chain CDR1 | Light chain CDR3 |
| Ab10 | WINPNSGGTNYAQKFQG | PAPRLRGAFDI | SGSSSNIGNKKVS | ATWDSSLRAVV |
| Ab10-1 | WINPNSSGTNYAQKFQG | | | |
| Ab10-2 | | LAPRLRGAFDI | | |
| Ab10-3 | | PAWRLRGAFDI | | |
| Ab10-4 | | PAPRLRMAFDI | | |
| Ab10-5 | | PAPRLRGAYDI | | |
| Ab10- 6 | | | SGSSSNIGTKKVS | |
| Ab10-7 | | | SGSSSNIGNFKVS | |
| Ab10-8 | | | | VTWDSSLRAVV |

The amino acids underlined correspond to residues mutated from Ab10, which is the parent antibody. CDR sequences which are not shown for a given mutant antibody are identical to those of Ab10, which is the parent antibody. For example, the Ab10-1 antibody is an antibody obtained by inducing a mutation only in the heavy chain CDR2, and the sequences of the remaining CDRs, that is, the sequences of the heavy chain CDR1, the heavy chain CDR3, the light chain CDR1, the light chain CDR2, and the light chain CDR3 are the same as those of Ab10. In addition, in order to prevent the post-translational modification of the antibody Ab10, a mutant (Ab10-10) in which serine as the 6th residue in CDR2 was replaced with threonine was produced, where the serine as the 6th residue was the same as in the mutant (Ab10-9) in which asparagine as the 5th residue in the heavy chain CDR2 was replaced with isoleucine.

**[Table 36]**

| CDR sequences of antibodies Ab10-9 and 10-10 | | | | |
|---|---|---|---|---|
| Antibody ID | Heavy chain CDR2 | Heavy chain CDR3 | Light chain CDR1 | Light chain CDR3 |
| Ab10 | WINPNSGGTNYAQKFQG | PAPRLRGAFDI | SGSSSNIGNKKVS | ATWDSSLRAVV |
| Ab10-9 | WINPISGGTNYAQKFQG | | | |
| Ab10-10 | WINPNTGGTNYAQKFQG | | | |

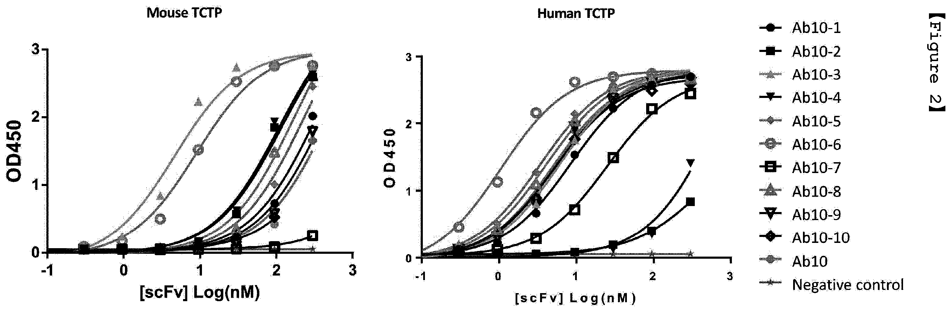

The amino acids underlined correspond to residues mutated from Ab10, which is the parent antibody. CDR sequences which are not shown for a given mutant antibody are identical to those of Ab10, which is the parent antibody. As a result of assaying the binding of these mutants to TCTP using ELISA, it could be confirmed that antibodies Ab10-1, Ab10-2, Ab10-3, Ab10-4, Ab10-5, Ab10-6, Ab10-7, Ab10-8 have increased binding capacity to mouse TCTP as compared with Ab10 which is the parent antibody, while having the same binding capacity to human TCTP as the antibody Ab10 which is the parent antibody (Fig. 2). In addition, in the case of Ab10-9 and Ab10-10, it was confirmed that the binding capacity to human TCTP was maintained equally even though the possibility of an acidic variant was low (Fig. 2). The mutants of the Ab10 antibody are mutants in which one amino acid is mutated from the Ab10 antibody, and these mutants can also be implemented in a form in which two or more mutations are simultaneously introduced.

### Example 4: Inhibition of binding of TCTP antibody between TCTP and TLR

### Example 4-1: ELISA

The biological function of TCTP outside cells is known through TLR2, which is a cell membrane receptor. ELISA analysis was carried out to check whether a selected TCTP antibody can inhibit binding between TCTP and TLR2.

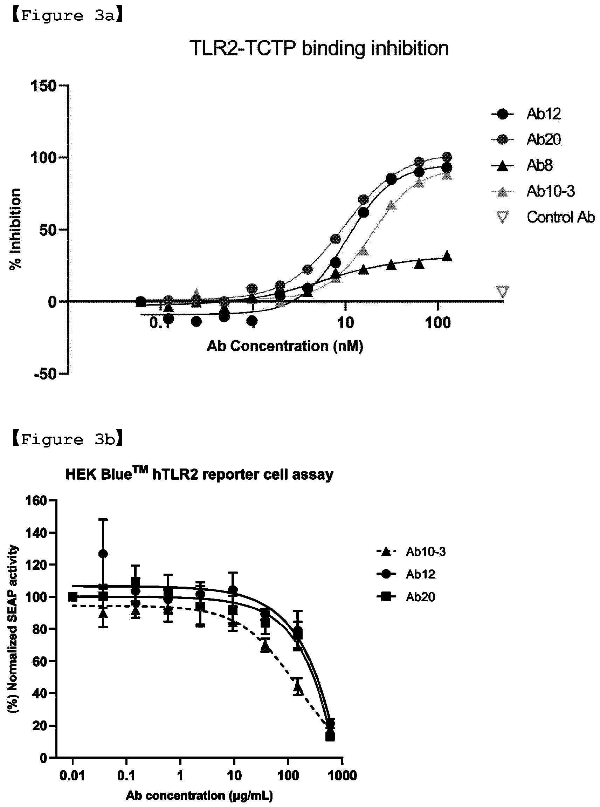

To this end, the binding inhibition between TCTP and TLR2 was checked by a method of attaching human TCTP to an ELISA plate at a concentration of 1 µg/ml, subsequently carrying out treatment with 150 nM of TLR2 and the antibodies Ab8, Ab10-3, Ab12, and Ab20 at various concentrations in each well, and then detecting TLR2 bound to human TCTP. As a result, it was confirmed that Ab10-3, Ab12, and Ab20 inhibit binding between TCTP and TLR2 (Fig. 3a).

### Example 4-2: Reporter cell assay

TLR2 is known to regulate immune cells through ligand binding, by using the NF-kappa B signal transduction. Using the antibodies that had been proven to inhibit TCTP-TLR2 binding In Example 4-1, an analysis was carried out using a HEK Blue^{™} hTLR2 reporter cell to check whether signal transduction is inhibited at the cellular level.

To this end, HEK Blue^{™} hTLR2 reporter cells were dispensed at 5 × 10⁴ cells per well of a 96-well plate and cultured, and then simultaneously treated with 9 µg/ml of human TCTP and the antibodies Ab10-3, Ab12, and Ab20 at various concentrations. Then, the activity of secreted alkaline phosphatase (SEAP) regulated by NF-kappa B was checked. As a result, it was confirmed that Ab10, Ab12, and Ab20 inhibits signals caused by TLR2 (Fig. 3b).

### Example 5: inhibitory effect of TCTP antibody on PMN-MDSC

TCTP has the effect of suppressing anti-cancer immunity in the tumor immune microenvironment by stimulating M-MDSC outside cells. One of the effects is to recruit PMN-MDSC in the tumor immune microenvironment. An experiment was carried out to check whether the anti-TCTP antibody according to the present invention inhibits the action of TCTP and has an effect of alleviating the anti-cancer immunosuppressive environment by suppressing the accumulation of PMN-MDSC in the tumor immune microenvironment.

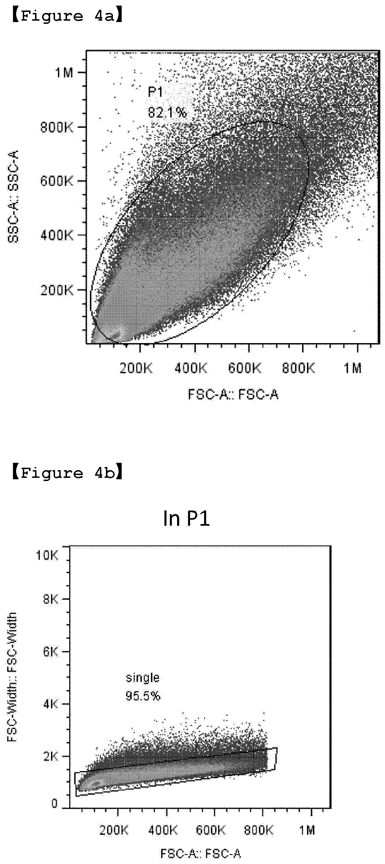

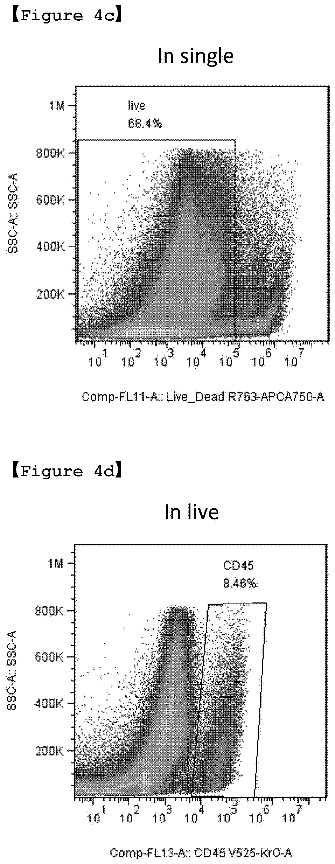

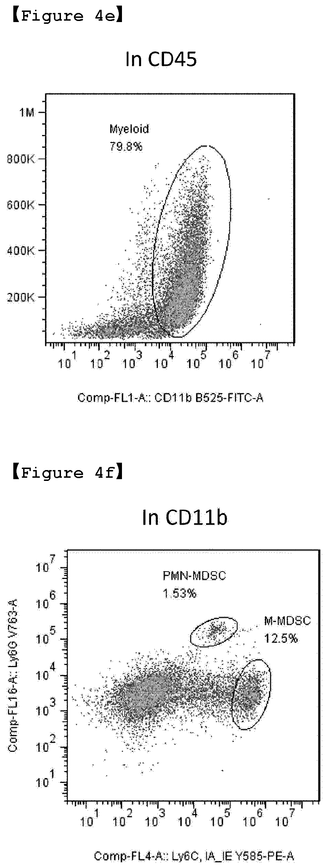

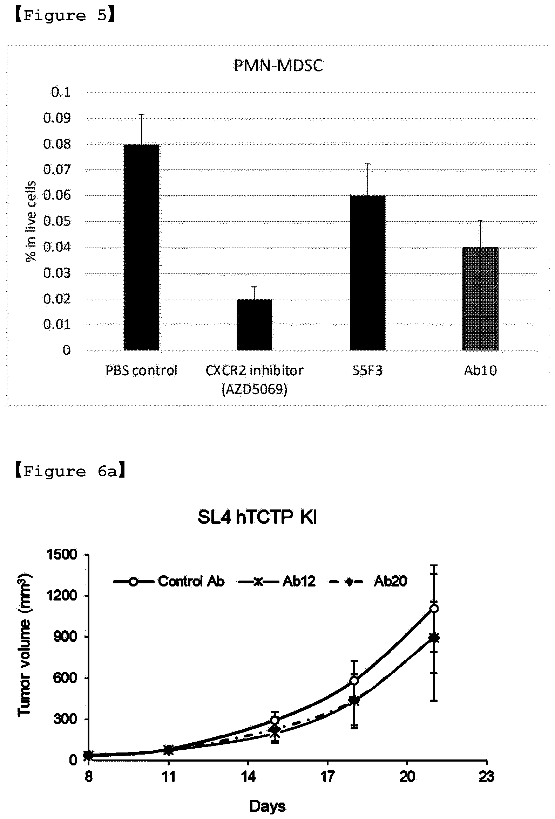

To this end, immune cells in the tumor immune microenvironment were collected from the HT29 cell line (human colon cancer cell line) tumors and analyzed by FACS. Tumor proliferation inhibition due to T cells may affect the accurate PMN-MDSC measurement. Therefore, The HT29 cell line (2 × 10⁶ cells) was subcutaneously transplanted into a BALB/c nude mouse without T cells. 13 days after transplantation, tumors were collected and separated into single cells, and then FACS analysis was carried out (Fig. 4a to Fig. 4f). For PMN-MDSC, the number of cells that were CD45+CD11b+LY6g+ were measured and evaluated. As shown in Fig. 5, it was confirmed that PMN-MDSC decreases in a case of being treated with a CXCR2 inhibitor (AZD5069) or anti-TCTP antibodies 55F3 and A10 in HT29 tumors. 55F3 is a publicly known anti-TCTP antibody. The CXCR2 inhibitor was used as a control group that suppresses the accumulation of PMN-MDSC.

### Example 6: Inhibitory effect of TCTP antibody on tumor proliferation

### Example 6-1: Single administration

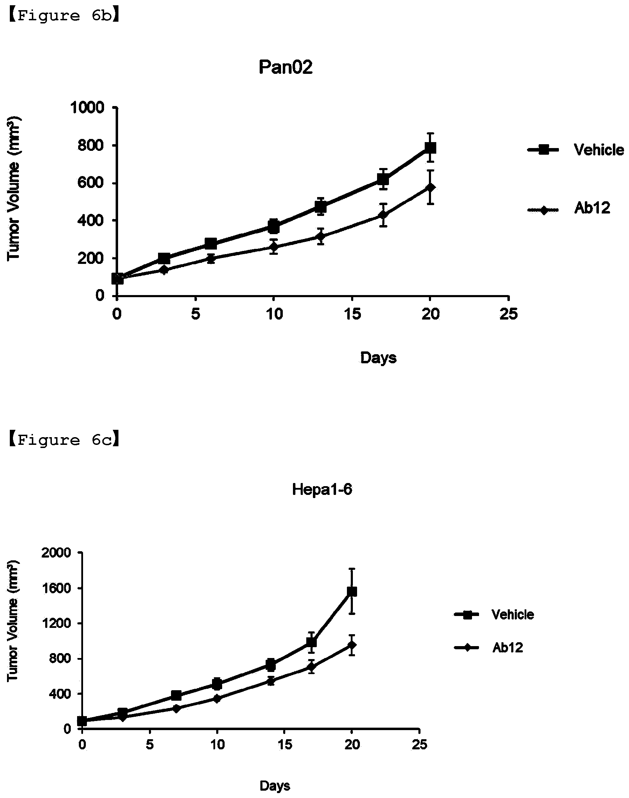

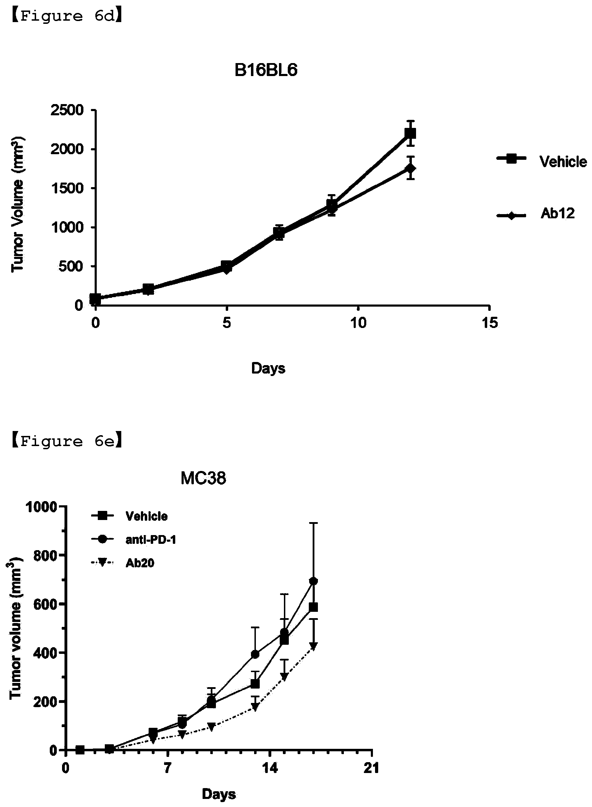

To check the tumor proliferation inhibitory activity of the anti-TCTP antibody, an SL4 cell line (SL4 hTCTP KI) which had been subjected to knock-in with human TCTP was used. SL4 hTCTP KI cells (2 x 10⁵ cells) were subcutaneously inoculated into a C57BL/6 mouse, and the anti-TCTP antibody (Ab12 or Ab20, 100 µg, intraperitoneal administration) or a control group isotype IgG antibody (BioXCell; 100 µg, intraperitoneal administration) was administered on Days 1, 4, 8, 11, and 15 based on the day of inoculation (Day 1). As a result, tumor growth inhibitory activity was confirmed in the animal model administered with Ab12 or Ab20 (Fig. 6a).

Mouse tumor cell lines Pan02 (pancreatic cancer), Hepa1-6 (liver cancer), B16BL6 (skin cancer), and MC38 (colon cancer) were subcutaneously inoculated into C57BL/6 mice (3 × 10⁵, 5 × 10⁵,2 × 10⁵, 1 ×10⁶ cells, respectively), and 100 µg of the antibodies Ab12, Ab20, or the anti-PD-1 antibody was administered intraperitoneally for 2 or 3 weeks based on the inoculation day (Day 1). As a result, it was confirmed that the growth rate of tumor proliferation was reduced by Ab12 or Ab20 in all four cancer cell lines (Figs. 6b to 6e). In an experiment using MC38, an anti-PD-1 antibody administration group was included, and it was found that Ab20 inhibits tumor growth better as compared with the anti-PD-1 antibody.

### Example 6-2: Administration in combination

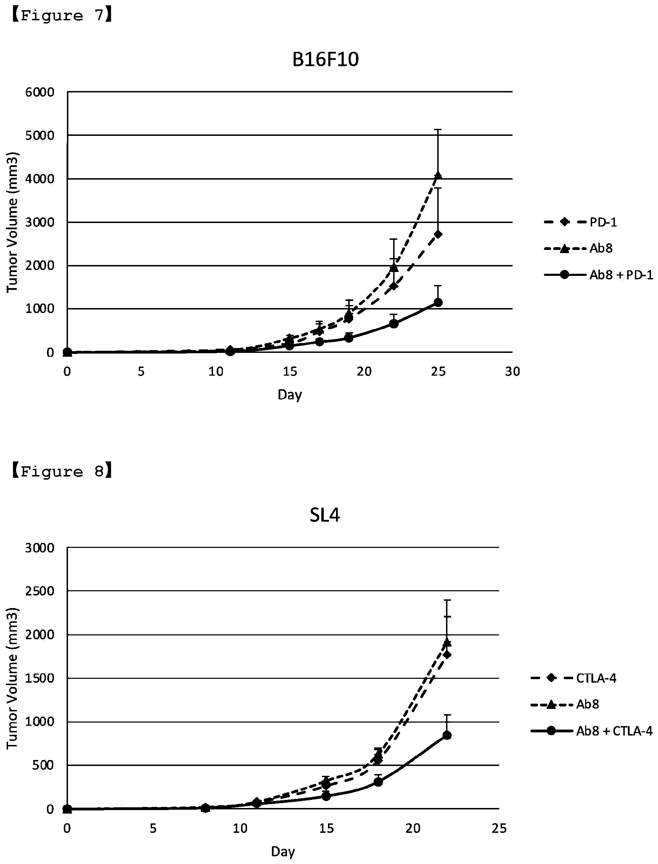

The effect of a combination of the anti-TCTP antibody and the anti-PD-1 antibody on tumor suppression was studied in order to check the effect of alleviating the immunosuppressive environment of the anti-TCTP antibody according to the present invention on the tumor proliferation in the tumor immune microenvironment. B16F10 (1 × 10⁵ cells) cells were subcutaneously inoculated into a C57BL/6 mouse. On Days 1, 4, 8, 11, 15, 18, and 22 based on the day of inoculation (Day 1), the anti-TCTP antibody Ab8 (100 µg, intraperitoneal administration) and an anti-PD-1 antibody (BioXCell; 100 µg, intravenous injection) were administered alone or in combination. As a result, it was confirmed that in a case where the anti-TCTP antibody was additionally administered in addition to the anti-PD-1 antibody, the tumor size was further reduced as compared with a case where only the anti-PD-1 antibody was administered (Fig. 7).

The same effect was also observed in another experiment of administration in combination with an immune checkpoint inhibitor using SL4 cells. SL4 cells (2 x 10⁵ cells) cells were subcutaneously inoculated into a C57BL/6 mouse. On Days 1, 4, 8, 11, 15, 18, 22 based on the day of inoculation (Day 1), the anti-TCTP antibody Ab8 (100 µg, intraperitoneal administration) and an anti-CTLA-4 antibody (BioXCell; 100 µg, intravenous injection) were administered alone or in combination. As a result, it was confirmed that in a case where the anti-TCTP antibody was additionally administered in addition to the anti-CTLA-4 antibody, the tumor size was further reduced as compared with a case where only the anti-CTLA-4 antibody was administered (Fig. 8). These results show that the TCTP-specific antibody according to the present invention not only effectively inhibits tumor proliferation by itself, but can also synergistically improve the inhibitory effect on tumor proliferation in a case of being used in combination with an immune checkpoint inhibitor.

## Claims

1. A binding molecule or a fragment thereof, which suppresses a function of a suppressive immune cell and/or activates an anti-cancer immune cell by binding to a human translationally controlled tumor protein (TCTP), a cleaved form thereof, or a multimer thereof with a dissociation constant (Kd) of about 1 × 10⁻⁷ or less in a case of being measured by surface plasmon resonance, and then suppressing a function thereof outside the cell.

2. A binding molecule or a fragment thereof, which specifically binds to TCTP, a post-translational variant thereof, a cleaved form thereof, or a multimer thereof, which contains heavy chain CDR1, heavy chain CDR2, heavy chain CDR3, light chain CDR1, light chain CDR2, and light chain CDR3 which are as defined in the following (a) to (f), respectively,
wherein (a) the heavy chain CDR1 comprises
(a1) an amino acid sequence of X1-X2-X3-X4-H (where X1 is S, G, or V, X2 is Y, H, or N, X3 is A or Y, and X4 is M or V),
(a2) an amino acid sequence of S-X5-X6-X7-X8-W-X9 (where X5 is S or N, X6 is N or S, X7 is A or W, X8 is A or is not present, and X9 is N or S),
(a3) an amino acid sequence of G-Y-T-F-T-G-Y-M-H, or
(a4) an amino acid sequence of N-A-R-M-G-V-S, or a conservative amino acid substituent thereof;
(b) the heavy chain CDR2 comprises
(b1) an amino acid sequence of E-I-X1-H-X2-G-X3-T-Y-N-P-S-L-K-S (where X1 is D or Y, X2 is S or is not present, and X3 is T or V),
(b2) an amino acid sequence of W-I-N-X4-X5-X6-X7-X8-T-X9-Y-X10-Q-K-F-Q-X11 (where X4 is A or P, X5 is G, I, or N, X6 is K, N, S, or T, X7 is G or S, X8 is G, N, Y, D, or K, X9 is K, N, or E, X10 is A or S, and X11 is D or G),
(b3) an amino acid sequence of R-T-X12-Y-X13-S-W-Y-N-D-Y-A-X14-S-V-K-S (where X12 is F or Y, X13 is K or R, and X14 is E or V), or
(b4) an amino acid sequence of H-I-F-S-N-D-E-K-S-Y-S-Y-S-L-K-S, or a conservative amino acid substituent thereof;
(c) the heavy chain CDR3 comprises
(c1) an amino acid sequence of G-X1-X2-X3-X4-F-D-X5 (where X1 is R or G, X2 is A or S, X3 is A or V, X4 is A or V, and X5 is P or Y),
(c2) an amino acid sequence of D-G-S-G-S-Y-E-G-Y,
(c3) an amino acid sequence of W-V-F-D-Y,
(c4) an amino acid sequence of X6-A-X7-R-L-X8-A-X9-D-I (where X6 is L or P, X7 is P or W, X8 is G or M, and X9 is F or Y),
(c5) an amino acid sequence of R-L-T-I-V-R-G-V-M-R-G-M-D-V,
(c6) an amino acid sequence of G-Y-Y-D-I-L-T-G-Y-T-T-D-A-F-D-I,
(c7) an amino acid sequence of X10-X11-X12-X13-X14-X15-X16-X17-X18-F-D-Y (where X10 is V or is not present, X11 is R or L or is not present, X12 is G or L, X13 is Y, I, T, or W, X14 is F or T, X15 is D or G, X16 is W, T, Y, or E, X17 is W, T, Y, or L, and X18 is A, P, or Y),
(c8) an amino acid sequence of G-G-V-L-Y-F-G-E-L-S-R-F-D-Y, or
(c9) an amino acid sequence of E-G-I-T-V-G-R-G-M-L-Y-F-D-L, or a conservative amino acid substituent thereof;
(d) the light chain CDR1 comprises
(d1) an amino acid sequence of R-A-S-Q-X1-X2-X3-X4-X5-L-X6 (where X1 is A, D, or G, X2 is I or V, X3 is S, R, or G, X4 is N or S, X5 is H, Y, D, N, or S, and X6 is A, G, or H),
(d2) an amino acid sequence of X7-G-X8-X9-S-X10-X11-G-X12-X13-X14-X15-V-S (where X7 is S or T, X8 is S or T, X9 is N or S, X10 is D or N, X11 is I or V, X12 is G, N, or T, X13 is F, K, or Y, X14 is N or is not present, and X15 is K or Y),
(d3) an amino acid sequence of K-S-S-Q-X16-X17-L-Y-X18-X19-N-N-K-X20-Y-X21-A (where X16 is N or S, X17 is I, L, or V, X18 is N or S, X19 is A or S, X20 is D or N, and X21 is I or L), or
(d4) an amino acid sequence of R-S-S-Q-S-V-X22-X23-D-G-N-T-X24-L-X25 (where X22 is H or Y, X23 is R or S, X24 is Y or H, and X25 is S, N, or K), or a conservative amino acid substituent thereof;
(e) the light chain CDR2 comprises
an amino acid sequence of X1-X2-X3-X4-X5-X6-X7 (where X1 is A, D, E, G, K, W, or Y, X2 is A, D, I, or V, X3 is N, S, or T, X4 is I, K, N, Q, S, or T, X5 is L, R, S, or W, X6 is A, D, F, G, K, P, Q, or Y, and X7 is F, S or T) or a conservative amino acid substituent thereof; and
(f) the light chain CDR3 comprises
(f1) an amino acid sequence of X1-Q-X2-X3-X4-X5-P-X6-T (where X1 is M or Q, X2 is A, G, or Y, X3 is F, H, N, S, or Y, X4 is G, H, Q, N, S, or Y, X5 is F, I, L, T, Y, or W, and X6 is H, I, L, P, R, W, or Y),
(f2) an amino acid sequence of X7-X8-X9-X10-X11-X12-X13-X14-X15-V (where X7 is A, N, or V, X8 is S or T, X9 is W or Y, X10 is A or D, X11 is G or S, X12 is D, N, or S, X13 is L or N, X14 is N or R, and X15 is A or L), or
(f3) an amino acid sequence of X16-Q-X17-X18-S-X19-P-X20-T (where X16 is L or H, X17 is H or S, X18 is N or S, X19 is Y or L, and X20 is Y, R, or Q), or a conservative amino acid substituent thereof.

3. The binding molecule or the fragment thereof according to claim 1 or claim 2 (preferably claim 2),
wherein the heavy chain CDR1 comprises the amino acid sequence set forth in SEQ ID NO: 1, 7, 13, 19, 25, 31, 37, 43, 49, 55, 61, 67, 73, 79, 85, 91, 97, 103, 109, 115, 115, or 121, or a conservative amino acid substituent thereof,
the heavy chain CDR2 comprises the amino acid sequence set forth in SEQ ID NO: 2, 8, 14, 20, 26, 32, 38, 44, 50, 56, 62, 68, 80, 76, 74, 74, 86, 92, 98, 104, 110, 116, 122, 127, 135, or 136, or a conservative amino acid substituent thereof,
the heavy chain CDR3 comprises the amino acid sequence set forth in SEQ ID NO: 3, 9, 15, 21, 27, 33, 39, 45, 51, 57, 63, 69, 75, 81, 87, 93, 99, 105, 111, 117, 123, 128, 129, 130, or 131, or a conservative amino acid substituent thereof,
the light chain CDR1 comprises the amino acid sequence set forth in SEQ ID NO: 4, 22, 16, 10, 16, 28, 34, 40, 46, 52, 58, 64, 70, 76, 82, 88, 94, 100, 106, 112, 118, 124, 132, or 133, or a conservative amino acid substituent thereof,
the light chain CDR2 comprises the amino acid sequence set forth in SEQ ID NO: 5, 11, 17, 23, 29, 35, 41, 47, 53, 59, 65, 71, 77, 83, 89, 95, 101, 107, 113, 119, or 125, or a conservative amino acid substituent thereof, and
the light chain CDR3 comprises the amino acid sequence set forth in SEQ ID NO: 6, 12, 18, 24, 30, 36, 42, 48, 54, 60, 66, 72, 78, 84, 90, 96, 102, 108, 114, 120, 126, or 134, or a conservative amino acid substituent thereof.

4. The binding molecule or the fragment thereof according to any one of claim 1 to claim 3 (preferably claim 2),
wherein the heavy chain variable region comprises the amino acid sequence selected from the group consisting of SEQ ID NOs: 137, 139, 141, 143, 145, 147, 149, 151, 153, 155, 157, 159, 161, 163, 165, 167, 169, 171, 173, 175, and 177, or the amino acid sequence that has a sequence homology of at least 90% thereto, and
the light chain variable region comprises the amino acid sequence selected from the group consisting of SEQ ID NOs:138, 140, 142, 144, 146, 148, 150, 152, 154, 156, 158, 160, 162, 164, 166, 168, 170, 172, 174, 176, and 178, or the amino acid sequence that has a sequence homology of at least 90% thereto.

5. The binding molecule or the fragment thereof according to any one of claim 1 to claim 4 (preferably claim 2),
wherein the binding molecule or the fragment thereof competes for the binding to human TCTP with the antibody comprising the heavy chain CDR1 comprising the amino acid sequence set forth in SEQ ID NO: 1, 7, 13, 19, 25, 31, 37, 43, 49, 55, 61, 67, 73, 79, 85, 91, 97, 103, 109, 115, 115, or 121, the heavy chain CDR2 comprising the amino acid sequence set forth in SEQ ID NO:2, 8, 14, 20, 26, 32, 38, 44, 50, 56, 62, 68, 74, 80, 86, 92, 98, 104, 110, 116, 122, 127, 135, or 136, the heavy chain CDR3 comprising the amino acid sequence set forth in SEQ ID NO: 3, 9, 15, 21, 27, 33, 39, 45, 51, 57, 63, 69, 75, 81, 87, 93, 99, 105, 111, 117, 123, 128, 129, 130, or 131, the light chain CDR1 comprising the amino acid sequence set forth in SEQ ID NO:4, 10, 16, 22, 28, 34, 40, 46, 52, 58, 64, 70, 76, 82, 88, 94, 100, 106, 112, 118, 124, 132, or 133, the light chain CDR2 comprising the amino acid sequence set forth in SEQ ID NO: 5, 11, 17, 23, 29, 35, 41, 47, 53, 59, 65, 71, 77, 83, 89, 95, 101, 107, 113, 119, or 125, and the light chain CDR3 comprising the amino acid sequence set forth in SEQ ID NO: 6, 12, 18, 24, 30, 36, 42, 48, 54, 60, 66, 72, 78, 84, 90, 96, 102, 108, 114, 120, 126, or 134.

6. The binding molecule or the fragment thereof according to any one of claim 1 to claim 5 (preferably claim 2),
wherein the binding molecule or the fragment thereof is an antibody or an antigen-binding fragment thereof comprising the heavy chain CDR1 comprising the amino acid sequence set forth in SEQ ID NO: 43, the heavy chain CDR2 comprising the amino acid sequence set forth in SEQ ID NO: 44, the heavy chain CDR3 comprising the amino acid sequence set forth in SEQ ID NO: 45, the light chain CDR1 comprising the amino acid sequence set forth in SEQ ID NO: 46, the light chain CDR2 comprising the amino acid sequence set forth in SEQ ID NO:47, and the light chain CDR3 comprising the amino acid sequence set forth in SEQ ID NO: 48.

7. The binding molecule or the fragment thereof according to according to any one of claim 1 to claim 5 (preferably claim 2),
wherein the binding molecule or the fragment thereof is an antibody or an antigen-binding fragment thereof comprising the heavy chain CDR1 comprising the amino acid sequence set forth in SEQ ID NO: 55, the heavy chain CDR2 comprising the amino acid sequence set forth in SEQ ID NO: 56, the heavy chain CDR3 comprising the amino acid sequence set forth in SEQ ID NO:57, the light chain CDR1 comprising the amino acid sequence set forth in SEQ ID NO: 58, the light chain CDR2 comprising the amino acid sequence set forth in SEQ ID NO:59, and the light chain CDR3 comprising the amino acid sequence set forth in SEQ ID NO: 60.

8. The binding molecule or the fragment thereof according to any one of claim 1 to claim 5 (preferably claim 2),
wherein the binding molecule or the fragment thereof is an antibody or an antigen-binding fragment thereof comprising the heavy chain CDR1 comprising the amino acid sequence set forth in SEQ ID NO: 61, the heavy chain CDR2 comprising the amino acid sequence set forth in SEQ ID NO: 62, the heavy chain CDR3 comprising the amino acid sequence set forth in SEQ ID NO: 63, the light chain CDR1 comprising the amino acid sequence set forth in SEQ ID NO: 64, the light chain CDR2 comprising the amino acid sequence set forth in SEQ ID NO: 65, and the light chain CDR3 comprising the amino acid sequence set forth in SEQ ID NO: 66.

9. The binding molecule or the fragment thereof according to any one of claim 1 to claim 5 (preferably claim 2),
wherein the binding molecule or the fragment thereof is an antibody or an antigen-binding fragment thereof comprising the heavy chain CDR1 comprising the amino acid sequence set forth in SEQ ID NO:109, the heavy chain CDR2 comprising the amino acid sequence set forth in SEQ ID NO: 110, the heavy chain CDR3 comprising the amino acid sequence set forth in SEQ ID NO: 111, the light chain CDR1 comprising the amino acid sequence set forth in SEQ ID NO: 112, the light chain CDR2 comprising the amino acid sequence set forth in SEQ ID NO: 113, and the light chain CDR3 comprising the amino acid sequence set forth in SEQ ID NO: 114.

10. The binding molecule or the fragment thereof according to any one of claim 1 to claim 9 (preferably claim 2),
wherein the binding molecule is an antibody, and the fragment thereof is an antigen-binding fragment.

11. An isolated polynucleotide that encodes the binding molecule or the fragment thereof according to any one of claim 1 to clam 9 (preferably claim 2).

12. A method of producing the binding molecule or the fragment thereof according to any one of claim 1 to claim 9 (preferably claim 2), the method comprising the steps of expressing, in a host cell, an isolated polynucleotide that encodes the binding molecule or the fragment thereof according to claim 2 and isolating the expressed binding molecule or fragment thereof.

13. A pharmaceutical composition for treating or preventing cancer, the pharmaceutical composition comprising the binding molecule or the fragment thereof according to any one of claim 1 to claim 9 (preferably claim 2).

14. A pharmaceutical composition for enhancing responsiveness to a cancer immunotherapeutic agent in an individual being required to be subjected to treatment for cancer, wherein the individual has no response or is likely to have no response to, or has a limited response or is likely to have a limited response to treatment using a cancer immunotherapeutic agent, and the pharmaceutical composition comprises the binding molecule or the fragment thereof according to any one of claim 1 to claim 9 (preferably claim 2).

15. A pharmaceutical composition for removing, alleviating, or preventing immune suppression in a tumor microenvironment of a cancer patient, the pharmaceutical composition comprising the binding molecule or the fragment thereof according to any one of claim 1 to claim 9 (preferably claim 2).

16. A pharmaceutical composition for suppressing a suppressive immune cell in a tumor microenvironment of a cancer patient, the pharmaceutical composition comprising the binding molecule or the fragment thereof according to any one of claim 1 to claim 9 (preferably claim 2).

17. The pharmaceutical composition according to Claim 16,
wherein the suppressive immune cell is a bone marrow-derived suppressor cell (MDSC).

18. The pharmaceutical composition according to any one of claim 13 to claim 17 (preferably claim 13),
wherein the cancer is selected from the group consisting of colorectal cancer, melanoma, fibrosarcoma, pancreatic cancer, liver cancer, and skin cancer.

19. The pharmaceutical composition according to any one of claim 13 to claim 17 (preferably claim 13),
wherein the pharmaceutical composition is used together with an immune checkpoint inhibitor.
